# EUROPÄISCHE PATENTSCHRIFT

(11) **EP 2 995 609 B1**
(45) Veröffentlichungstag und Bekanntmachung des Hinweises auf die Patenterteilung: **31.01.2018**
(21) Anmeldenummer: 14184099.1
(22) Anmeldetag: 09.09.2014
(51) Int. Cl.: C07C 217/28, C07C 43/178, C08G 18/48, C08G 18/62, C08G 18/75, C08G 59/14, C08G 59/22, C08G 18/10

(54) **Alkenyletherpolyole**
Alkenyl ether polyols
Polyols d'éther alcényliques

(43) Veröffentlichungstag der Anmeldung: 16.03.2016
(73) Patentinhaber: Henkel AG & Co. KGaA, 40589 Düsseldorf (DE); Max-Planck-Gesellschaft zur Förderung der Wissenschaften e.V., 80539 München (DE)
(72) Erfinder: Taden, Andreas, 40597 Düsseldorf (DE); Landfester, Katharina, 55122 Mainz (DE); Kirschbaum, Stefan, 51379 Leverkusen (DE)

(56) Entgegenhaltungen:
- WO-A1-2012/113618
- JP-A- H07 224 131
- RU-C1- 2 100 377
- US-A- 4 485 211
- US-A1- 2012 035 381
- MARCO SANGERMANO ET AL: "Cationic UV-Curing: Technology and Applications", MACROMOLECULAR MATERIALS AND ENGINEERING, Bd. 299, Nr. 7, 14. Juli 2014 (2014-07-14) , Seiten 775-793, XP55168964, ISSN: 1438-7492, DOI: 10.1002/mame.201300349

## Beschreibung

Die vorliegende Erfindung betrifft ein Verfahren zur Herstellung von Alkenyletherpolyolen und richtet sich darüber hinaus auf Alkenyletherpolyole, die mittels des erfindungsgemäßen Verfahrens erhältlich sind, sowie deren Verwendung zur Synthese von strahlenvernetzbaren Oligomeren oder Polymeren mittels Polyadditions- oder Polykondensationsreaktionen, insbesondere zur Synthese von UV-härtbaren Polyestern, Polyethern, Polyurethanen und Polyharnstoffen, sowie die derart erhältlichen UV-härtbaren Polymere.

Kationische Polymerisierungsverfahren, insbesondere initiiert durch hochenergetische Strahlung wie beispielsweise ultraviolette elektromagnetische Strahlung (UV) oder Elektronenstrahlung (EB), sind im Stand der Technik bekannt und finden vermehrt Anwendung in industriellen Herstellungsprozessen einer Vielzahl von Produkten, darunter Kleb- und Leimstoffe, Beschichtungszusammensetzungen und viele mehr. Des Weiteren sind niederenergetische Initiierungsmethoden für kationische Polymerisation bekannt geworden, beispielsweise basierend auf sichtbarem Licht. Letztgenannte Systeme finden zurzeit weniger Verwendung und befinden sich teilweise noch in der Entwicklung. Kationische Polymerisationsverfahren sind typischerweise frei von Lösemitteln, energieeffizient, umweltfreundlich und geeignet für automatisierte Prozessabläufe. Ein ausführlicher Überblick über kationische Polymerisierungsverfahren findet sich in Matyjaszewski, K. Hrsg., Cationic Polymerizations: Mechanisms, Synthesis, and Applications. (In: Plast. Eng. (N. Y.), 1996; 35, Dekker). Eine Übersicht über UV-härtende kationische Polymerisation geben beispielsweise M. Sangermano, N. Razza und J. V. Crivello (Macromol. Mater. Eng., 2014, 299, S. 775-793). Zu den gemeinhin anerkannten Vorteilen der kationischen Polymerisation zählen insbesondere die Tolerierung von (Luft-)Sauerstoff sowie die Möglichkeit einer fortlaufenden Reaktion nach Initiierung. In der einschlägigen Literatur wird dieses Verhalten auch als "Dark Cure" bezeichnet, und umschreibt das fortlaufende Aushärten des polymerisierbaren Materials nach erfolgter Stoßinitiierung, beispielsweise durch UV, EB oder Hitzeschlag, auch in Abwesenheit von elektromagnetischer Strahlung, Elektronenstrahlen oder erhöhter Wärmezufuhr. Dieses Dark-Cure-Verhalten ist besonders vorteilhaft für eine Vielzahl von technischen Fügeprozessen.

Generell konkurrieren diese Verfahren mit radikalischen Kettenpolymerisationsverfahren initiiert durch elektromagnetische oder EB-Strahlung, deren Durchsatz, und damit einhergehend Anwendbarkeit, unter der Sauerstoff-empfindlichen Natur des radikalischen Polymerisationsmechanismus leidet.

In Bezug auf kationische Polymerisation sind Alkenylether für ihre hohe Reaktivität bekannt und dienen daher als monomere Vorstufen. Die Alkenylether-funktionalisierten monomeren Vorstufen haben eine Reihe von Vorteilen: aufgrund ihrer hohen Reaktivität härten sie schnell aus, sind eher geruchsneutral und weisen gute Haltbarkeits- und Haftungseigenschaften auf. Durch die hohe Reaktivität können sie darüber hinaus eine Vielzahl von funktionellen Gruppen weitgehend tolerieren, wie z.B. Urethangruppen, und werden weniger leicht durch Nebenreaktionen in der Polymerisation gestört.

Auch Epoxide und Oxetane sind geeignete reaktive Vorstufen für kationische Polymerisierungsverfahren, benötigen allerdings oftmals einen thermischen "Schub" nach der eigentlichen Initialisierung, um Polymerisierungsrate und Umsatz zu erhöhen. Zwar zählen auch Epoxide und Oxetane zu den reaktivsten kationisch polymerisierbaren Verbindungen, doch wird die Reaktion durch eine größere Vielfalt von funktionellen Gruppen gestört oder sogar zum Erliegen gebracht. So ist bekannt, dass bei Epoxiden und Oxetanen eine kationische Polymerisation bzw. Vernetzung in Gegenwart von Urethangruppen frühzeitig zum Erliegen neigt.

Generell gilt zu beachten, dass niedermolekulare (strahlenhärtende) Verbindungen, ganz gleich ob radikalisch und/oder kationisch polymerisierbar, für viele Anwendungen nicht in Frage kommen oder aber deutliche Nachteile aufweisen. Durch das niedrige Molekulargewicht weisen solche Verbindungen oft schon bei niedrigen Temperaturen einen merklichen Dampfdruck auf und können somit als "volatile organic compounds" (VOC) wirken. Die Flüchtigkeit der Substanzen ist problematisch für die Kontamination der Umwelt (Emissionen durch Verdampfen) sowie für unterschiedliche gesundheitliche und sicherheitstechnische Belange (Resorption über Atemwege, Haut, Verdauung, etc./ Bildung zündfähiger Gemische / etc.). Eine weitere wesentliche Einschränkung ist die mangelnde Einstellbarkeit der rheologischen oder mechanischen Eigenschaften solcher niedermolekularer Materialien vor der eigentlichen Härtung.

Aus diesem Grund werden im Stand der Technik üblicherweise Oligomere oder Polymere eingesetzt, welche die Problematiken, hervorgerufen durch Dampfdruck oder Applikationsviskosität, weitgehend umgehen können. Weit verbreitet sind in diesem Zusammenhang Polyurethane bzw. Polyurethan-Präpolymere (PU), die sich über einen weiten Eigenschaftsbereich einstellen lassen, und durch radikalisch reaktive Gruppen funktionalisiert sind. Am gängigsten ist dabei eine, vorzugsweise terminale, Funktionalisierung mittels Acrylat- oder Methacrylatgruppen. Die entsprechenden Materialien werden häufig in Kurzform als PUA oder PUMA bezeichnet. Nachteilig bei diesen Verbindungen ist die prinzipiell bedingte luft- bzw. sauerstoffempfindliche Vernetzungsreaktion. Als weiterhin nachteilig können geringe Vernetzungsgrade angeführt werden, insbesondere bei Verwendung von linearen, endständig funktionalisierten PUA- oder PUMA-Verbindungen.

Des Weiteren sind im Stand der Technik endständig funktionalisierte Alkylenether-funktionalisierte Oligomere oder Polymere bekannt, die sich kationisch polymerisieren lassen und keiner Sauerstoffinhibierung unterliegen. Die entsprechenden vollständig auspolymerisierten Systeme zeigen eine eher geringe Quervernetzungsdichte, da typischerweise ausschließlich die endständigen Gruppen miteinander reagieren. Die mechanischen Eigenschaften des gewünschten Polymers, beziehungsweise des entsprechenden Polymer-haltigen Produktes können daher nur bedingt und innerhalb eines gewissen Rahmens beeinflusst werden.

Es besteht folglich Bedarf an einem gegenüber dem Stand der Technik verbesserten System, das durch kationische Polymerisation hohe Vernetzungsdichten liefern kann. Dieses System soll zudem einen kaum merklichen Dampfdruck aufweisen, also nicht unter die gängigen VOC-Richtlinien fallen, sowie prinzipiell über einen weiten Bereich die Einstellung von rheologischen und mechanischen Eigenschaften bereits vor der kationischen Polymerisation erlauben und somit eine Anpassung an eine Vielzahl an Anwendungen erlauben.

Durch eine zu erzielende hohe Quervernetzungsdichte können die mechanischen Eigenschaften der entsprechenden Polymere beziehungsweise der Polymer-haltigen Produkte, beispielsweise Kleb- und Leimstoffe, sowie Beschichtungszusammensetzungen, dahingehend beeinflusst werden, dass sie, je nach Bedarf, mehr oder weniger drastisch von den mechanischen Eigenschaften der verwendeten Präpolymere abweichen.

Es wurde nun gefunden, dass Alkenylether-funktionalisierte Polyole hervorragende Vorstufen für zahlreiche kationisch polymerisierbare Verbindungen darstellen, die insbesondere über Polykondensations- und Polyadditionsreaktionen hergestellt werden können. Diese kationisch polymerisierbaren Verbindungen erlauben wiederum je nach Struktur und Funktionalisierungsgrad eine gute Kontrolle über die Quervernetzungsdichte in den resultierenden polymeren Systemen nach kationischer Aushärtung.

Die erfindungsgemäß hergestellten Alkenyletherpolyole können als Ausgangsstoffe für die Synthese von Oligomeren und Polymeren dienen, die über Polyadditionsverfahren oder Polykondensationsreaktionen zugänglich sind. Derart erhältliche Polymere schließen beispielsweise Polyester, Polyether, Polyurethane und Polyharnstoffe ein. Die Alkenyletherfunktionalitäten ermöglichen weitere Funktionalisierungs-, Quervernetzungs- und Polymerisationsreaktionen, beispielsweise kationische Polymerisation oder auch radikalische Copolymerisation, der Polyole und ihrer Reaktionsprodukte. Als weitere Beispiele seinen Thiol-ene Addition oder auch Acetal Bildung genannt.

Insbesondere ermöglichen die erfindungsgemäß hergestellten Alkenylether-funktionalisierten Polyole die Synthese neuartiger Polyurethanstrukturen, die in nachfolgenden kationischen Polymerisationsmechanismen unter Einwirkung elektromagnetischer Strahlung, insbesondere UV-Strahlung, oder EB-Einwirkung quervernetzt werden können. In der folgenden Beschreibung wird nicht weiter explizit zwischen elektromagnetischer Strahlung oder EB-Strahlung differenziert, sondern der Begriff UV als Synonym für zur Initiierung befähigte Strahlung verwendet. Es ist aber selbstverständlich, dass in den beschriebenen Ausführungsformen andere zur Initiierung fähige Strahlung anstelle von UV-Strahlung verwendet werden kann. Derartige Ausführungsformen fallen ebenfalls in den Umfang der vorliegenden Erfindung.

Ein erster Gegenstand der vorliegenden Erfindung ist daher ein Verfahren zur Herstellung eines Alkenyletherpolyols enthaltend mindestens eine Alkenylethergruppe, insbesondere eine 1-Alkenylethergruppe, und mindestens zwei Hydroxylgruppen (-OH), durch
A) Umsetzen eines Alkenylethers, enthaltend mindestens eine Alkenylethergruppe und mindestens eine funktionelle Gruppe ausgewählt aus -OH, -COOH, -SH, -NH₂ und deren Derivaten,
   mit (i) einem Epoxid oder (ii) einem cyclischen Carbonat oder Derivat davon; oder
B) Umsetzen eines Alkenylethers, enthaltend mindestens eine Alkenylethergruppe und mindestens eine funktionelle Gruppe ausgewählt aus (i) Epoxidgruppen und (ii) cyclischen Carbonatgruppen oder Derivaten davon,
   mit einem Alkohol, Thiol, einer Carbonsäure oder einem Amin oder Derivaten der vorgenannten.

Des Weiteren richtet sich die vorliegende Erfindung auf Alkenyletherpolyole der Formel (I) oder (V) 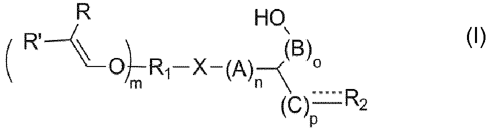 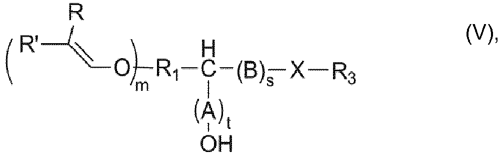 wobei
R₁ ein, mindestens 2-wertiges lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom,
R₂ ein optional 2- oder mehrwertiges, lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom, optional mit mindestens einer-OH Gruppe,
R₃ ein, optional 2- oder mehrwertiges, lineares oder verzweigtes, substituiertes oder unsubstituiertes Alkyl mit 1 bis 20 Kohlenstoffatomen, lineares oder verzweigtes, substituiertes oder unsubstituiertes Heteroalkyl mit 1 bis 20 Kohlenstoffatomen und mindestens einem Sauerstoff- oder Stickstoffatom, oder ein (Poly)Alkylenglykol der Formel -O-[CHRₐCH₂O]_{b}-R_{b} ist, wobei Rₐ H oder ein C₁₋₄-Alkylrest, R_{b} -H oder 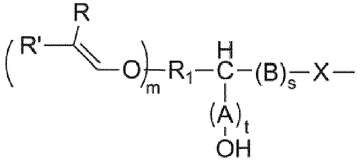
und b 1 bis 100 ist,
in Formel (I) X für O, S, C(=O)O, OC(=O)O, C(=O)OC(=O)O, NRₓ, NRₓC(=O)O, NRₓC(=O)NRₓ oder OC(=O)NRₓ steht,
in Formel (V) X für O, S, OC(=O), OC(=O)O, OC(=O)OC(=O), NR_{z}, NR_{z}C(=O)O, NR_{z}C(=O)NR_{z} oder OC(=O)NR_{z} steht,
jedes R und R' unabhängig ausgewählt ist aus H, C₁₋₂₀ Alkyl und C₂₋₂₀ Alkenyl, wobei insbesondere eines von R und R' H und das andere C₁₋₄ Alkyl oder beide H sind,
jedes A, B und C unabhängig ausgewählt ist aus CR"R''',
R" und R''' unabhängig ausgewählt sind aus H, einer funktionellen Gruppe, wie beispielsweise -OH, - NH₂, -NO₂, -CN, -OCN, -SCN, -NCO, -SCH, -SH, -SO₃H oder -SO₂H, und einem organischen Rest, insbesondere H und C₁₋₂₀ Alkyl, oder R" und R''' gemeinsam oder mit dem Kohlenstoffatom an welches sie gebunden sind ein organischer Rest sind, oder zwei von R" und R''' die an benachbarte Kohlenstoffatome gebunden sind, zusammen eine Bindung bilden um eine Doppelbindung zwischen den benachbarten Kohlenstoffatomen auszubilden,
----- eine Einfach- oder Doppelbindung ist, wobei wenn es eine Doppelbindung ist, das Kohlenstoffatom, das an R₂ gebunden ist nur einen Substituenten R" oder R''' trägt,
m eine ganze Zahl von 1 bis 10, vorzugsweise 1, ist,
n, p und o jeweils 0 oder eine ganze Zahl von 1 bis 10 sind, wobei n+p+o=1 oder mehr ist, insbesondere 1 oder 2,
s und t jeweils 0 oder eine ganze Zahl von 1 bis 10 sind, wobei s+t=1 oder mehr ist, insbesondere 1 oder 2,
Rₓ H oder 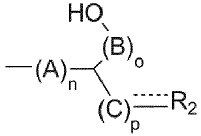 wobei wenn X nicht NRₓ mit Rₓ = 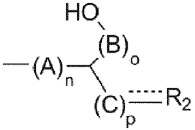 ist, R₂ mindestens einen Substituenten aufweist, der ausgewählt ist aus -OH und 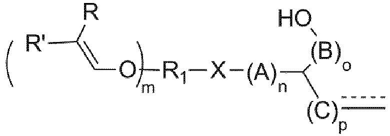 und
R_{z} H oder 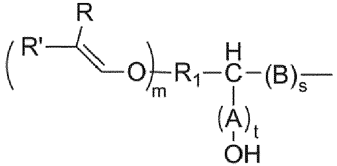
wobei wenn X nicht NR_{z} mit R_{z} = 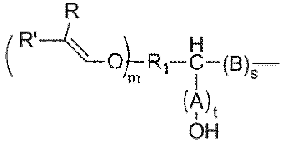 ist,
R₃ mindestens einen Substituenten aufweist, der ausgewählt ist aus -OH und

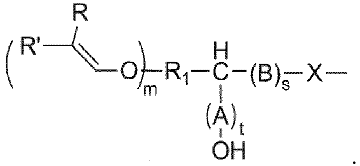

Die vorliegende Erfindung richtet sich darüber hinaus auf Alkenyletherpolyole, insbesondere solche der Formel (I) oder (V), die erhältlich sind nach einem Verfahren gemäß der vorliegenden Erfindung.

Ein weiterer Aspekt der Erfindung betrifft die Verwendung der hierin beschriebenen Alkenyletherpolyole zur Synthese von UV-vernetzbaren Oligomeren oder Polymeren mittels Polyadditions- oder Polykondensationsreaktionen, insbesondere zur Synthese von UV-härtbaren Polyestern, Polyethern, Polyurethanen und Polyharnstoffen, besonders bevorzugt UV-härtbaren Polyurethanen bzw. entsprechende Verfahren.

Schließlich ist ein weiterer Aspekt der vorliegenden Erfindung ein UV-härtbares Polymer erhältlich gemäß der vorstehend beschriebenen Verwendung/Verfahren, insbesondere ein UV-härtbares Polyurethanpolymer, welches durch Umsetzen mindestens eines Alkenyletherpolyols der vorliegenden Erfindung mit einem Polyisocyanat erhältlich ist.

"Alkenyletherpolyol", wie hierin verwendet, bezeichnet Verbindungen, die mindestens eine Gruppe der Formel -O-Alkenyl und mindestens zwei Hydroxygruppen enthalten. Es ist bevorzugt, dass das Alkenyletherpolyol einen organischen Rest umfasst, an den sowohl die Alkenylethergruppe als auch die Hydroxygruppen gebunden sind, d.h. die Hydroxygruppen nicht an die Alkenylgruppe gebunden sind. Es ist ferner bevorzugt, dass die Alkenylethergruppe eine 1-Alkenylethergruppe ist, d.h. die C-C-Doppelbindung benachbart zu dem Sauerstoffatom liegt.

Der Begriff "Alkyl", wie hierin verwendet, bezeichnet einen linearen oder verzweigten, unsubstituierten oder substituierten gesättigten Kohlenwasserstoffrest, insbesondere Reste der Formel CₙH₂ₙ₊₁.

Beispiele für Alkylreste schließen ein, ohne darauf beschränkt zu sein, Methyl, Ethyl, n-Propyl, isoPropyl, n-Butyl, 2-Butyl, tert-Butyl, n-Pentyl, n-Hexyl und ähnliche. "Heteroalkyl", wie hierin verwendet, bezeichnet Alkylreste, in denen mindestens ein Kohlenstoffatom durch ein Heteroatom, wie insbesondere Sauerstoff, Stickstoff oder Schwefel, ersetzt ist. Beispiele schließen, ohne Beschränkung, Ether und Polyether ein, beispielsweise Diethylether, Polyethylenoxid, Polypropylenoxid oder Polytetramethylenoxid.

Der Begriff "Alkenyl", wie hierin verwendet, bezeichnet einen linearen oder verzweigten, unsubstituierten oder substituierten Kohlenwasserstoffrest, der mindestens eine C-C Doppelbindung enthält.

"Substituiert", wie hierin insbesondere im Zusammenhang mit Alkyl- und Heteroalkylgruppen verwendet, bezieht sich auf Verbindungen, in denen ein oder mehrere Kohlenstoff- und/oder Wasserstoffatome durch andere Atome oder Gruppen ersetzt sind. Geeignete Substituenten schließen ein, ohne darauf beschränkt zu sein, -OH, -NH₂, -NO₂, -CN, -OCN, -SCN, -NCO, -SCH, -SH, -SO₃H, -SO₂H, -COOH, -CHO und ähnliche.

Der Begriff "organischer Rest", wie hierin verwendet, bezieht sich auf jeden organischen Rest, der Kohlenstoffatome enthält. Organische Reste können insbesondere von Kohlenwasserstoffen abgeleitet sein, wobei beliebige Kohlenstoff- und Wasserstoffatome durch andere Atome oder Gruppen ersetzt sein können. Organische Reste im Sinne der Erfindung enthalten in verschiedenen Ausführungsformen 1 bis 1000 Kohlenstoffatome.

"Epoxid", wie hierin verwendet, bezeichnet Verbindungen, die eine Epoxidgruppe enthalten.

"Cyclisches Carbonat", wie hierin verwendet, bezeichnet ringförmige Verbindungen, die als Ringbestandteil die Gruppe -O-C(=O)-O- enthalten.

Der Begriff "Alkohol" bezeichnet eine organische Verbindung, die mindestens eine Hydroxylgruppe (-OH) enthält.

Der Begriff "Amin" bezeichnet eine organische Verbindung, die mindestens eine primäre oder sekundäre Aminogruppe (-NH₂, -NHR) umfasst.

Der Begriff "Thiol" oder "Mercaptan" bezeichnet eine organische Verbindung, die mindestens eine Thiolgruppe (-SH) enthält.

Der Begriff "Carbonsäure" bezeichnet eine Verbindung, die mindestens eine Carboxylgruppe (-C(=O)OH) enthält.

Der Begriff "Derivat", wie hierin verwendet, bezeichnet eine chemische Verbindung, die gegenüber einer Referenzverbindung durch eine oder mehrerer chemischer Reaktionen verändert ist. Im Zusammenhang mit den funktionellen Gruppen -OH, -COOH, -SH und -NH₂ bzw. den Verbindungsklassen der Alkohole, Carbonsäuren, Thiole und Amine umfasst der Begriff "Derivat" insbesondere die korrespondierenden ionischen Gruppen/Verbindungen und deren Salze, d.h. Alkoholate, Carboxylate, Thiolate und Ammonium(quaternären Stickstoff-)verbindungen. Im Zusammenhang mit den cyclischen Carbonaten, umfasst der Begriff Derivat insbesondere die unten genauer beschriebenen Thioderivate der Carbonate, d.h. Verbindungen in den ein, zwei oder alle drei Sauerstoffatome der Gruppierung -O-C(=O)-O- durch Schwefelatome ersetzt sind.

"Mindestens", wie hierin im Zusammenhang mit einem Zahlenwert verwendet, bezieht sich auf genau diesen Zahlenwert oder mehr. "Mindestens ein" bedeutet somit 1 oder mehr, d.h. beispielsweise 2, 3, 4, 5, 6, 7, 8, 9, 10 oder mehr. Im Zusammenhang mit einer Art von Verbindung bezieht sich der Begriff nicht auf die absolute Anzahl der Moleküle, sondern vielmehr auf die Anzahl der Arten von Stoffen, die unter den jeweiligen Oberbegriff fallen. "Mindestens ein Epoxid" bedeutet somit beispielsweise, dass mindestens eine Art von Epoxid, aber auch mehrere verschiedene Epoxide enthalten sein können.

Der Begriff "härtbar", wie hierin verwendet, bezeichnet eine Veränderung des Zustandes und/oder der Struktur in einem Material durch chemische Reaktion, die für gewöhnlich, aber nicht zwingend, durch mindestens eine Variable, wie die Zeit, Temperatur, Feuchtigkeit, Strahlung, Anwesenheit und Quantität eines härtungs-vermittelnden Katalysators oder Beschleunigers und dergleichen induziert wird. Der Begriff bezieht sich sowohl auf das komplette wie auch partielle Aushärten des Materials. "UV-härtbar" oder "UV-vernetzbar", bezeichnet somit Verbindungen, die bei UV-Exposition chemisch reagieren und neue Bindungen (intra- oder intermolekular) ausbilden.

Der Begriff "zweiwertig" oder "2-wertig", wie hierin im Zusammenhang mit Resten oder Gruppen verwendet, bezeichnet einen Rest oder eine Gruppe, die mindestens zwei Anknüpfungsstellen hat, die eine Verbindung zu weiteren Molekülteilen herstellen. Ein zweiwertiger Alkylrest bedeutet somit im Sinne der vorliegenden Erfindung einen Rest der Formel -Alkyl-. Ein solcher zweiwertiger Alkylrest wird hierin auch als Alkylenylrest bezeichnet. "Mehrwertig" bedeutet dementsprechend, dass ein Rest oder einer Gruppe mehr als einen Anknüpfungspunkt besitzt. Beispielsweise kann ein solcher Rest auch drei-, vier-, fünf- oder sechswertig sein. "Mindestens 2-wertig" bedeutet somit 2- oder höherwertig.

Der Begriff "Poly-" bezieht sich auf eine sich wiederholende Einheit einer diesem Präfix nachgestellten (funktionellen) Gruppe oder strukturellen Einheit. So bezeichnet ein Polyol eine Verbindung mit mindestens 2 Hydroxygruppen, ein Polyalkylenglykol bezeichnet ein Polymer aus Alkylenglykol-Monomereinheiten.

"Polyisocyanat", wie hierin verwendet, bezieht sich auf organische Verbindungen, die mehr als eine Isocyanatgruppe (-NCO) enthalten.

Bei den Alkenylethern kann es sich um aliphatische Verbindungen handeln, welche außer der oder den Alkenylethergruppe(n) mindestens eine andere funktionelle Gruppen enthalten, die reaktiv gegenüber Epoxy bzw. Cyclocarbonatgruppen ist, darunter -OH, -COOH, -SH, -NH₂ und deren Derivate. Die funktionellen Gruppen greifen nucleophil am Ringkohlenstoff des Epoxidrings oder am Carbonyl-Kohlenstoffatom des Cyclocarbonats an, wobei sich der Ring öffnet und eine Hydroxylgruppe entsteht. In Abhängigkeit der reaktiven, nucleophilen Gruppe wird dabei, eine O-C-, N-C, S-C, oder O-/N-/S-C(=O)O-Bindung geknüpft.

Gemäß den hierin beschriebenen Verfahren kann die Herstellung des Alkenyletherpolyols über zwei alternative Routen A) und B) realisiert werden.

Bei Route A) wird ein Alkenylether, der mindestens eine Alkenylethergruppe und mindestens eine funktionelle Gruppe ausgewählt aus -OH, -COOH, -SH, -NH₂ und deren Derivaten enthält, mit (i) einem Epoxid oder (ii) einem cyclischen Carbonat oder Derivat davon umgesetzt.

Bei Route B) wird ein Alkenylether, der mindestens eine Alkenylethergruppe und mindestens eine funktionelle Gruppe ausgewählt aus (i) Epoxidgruppen und (ii) cyclischen Carbonatgruppen oder Derivaten davon enthält, mit einem Alkohol, Thiol, einer Carbonsäure oder einem Amin oder Derivaten der vorgenannten umgesetzt.

Unabhängig von der Route entstehen die Alkenyletherpolyole durch Reaktion der Hydroxy-, Thiol-, Carboxyl- oder Aminogruppen mit einer Epoxid- oder cyclischen Carbonatgruppe unter Ringöffnung.

In allen Ausführungsformen der Erfindung werden die Reaktionspartner derart ausgewählt, dass das Reaktionsprodukt, d.h. das erhaltene Alkenyletherpolyol mindestens zwei Hydroxylgruppen trägt.

In verschiedenen Ausführungsformen wird das Alkenyletherpolyol hergestellt durch Umsetzen eines Alkenylethers, enthaltend mindestens eine Alkenylethergruppe und mindestens eine funktionelle Gruppe ausgewählt aus -OH, -COOH, -SH, -NH₂ und deren Derivaten, mit (i) einem Epoxid oder (ii) einem cyclischen Carbonat oder Derivat davon, wobei das derart hergestellte Alkenyletherpolyol ein Alkenyletherpolyol der Formel (I) ist

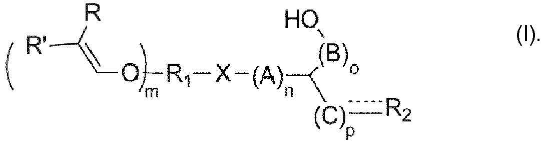

In den Verbindungen der Formel (I) ist
R₁ ein mindestens 2-wertiges lineares oder verzweigtes, substituiertes oder unsubstituiertes Alkyl mit 1 bis 50, vorzugsweise 1 bis 20 Kohlenstoffatomen, oder ein mindestens 2-wertiges lineares oder verzweigtes, substituiertes oder unsubstituiertes Heteroalkyl mit 1 bis 50, vorzugsweise 1 bis 20 Kohlenstoffatomen, und mindestens einem Sauerstoff- oder Stickstoffatom,
R₂ ein (optional 2- oder mehrwertiges) lineares oder verzweigtes, substituiertes oder unsubstituiertes Alkyl mit 1 bis 50, vorzugsweise 1 bis 20 Kohlenstoffatomen oder ein (optional 2- oder mehrwertiges) lineares oder verzweigtes, substituiertes oder unsubstituiertes Heteroalkyl mit 1 bis 50, vorzugsweise 1 bis 20 Kohlenstoffatomen und mindestens einem Sauerstoff- oder Stickstoffatom, optional mit mindestens einer -OH Gruppe. R₂ kann aber auch ein hochmolekularer Rest, wie beispielweise ein Polyalkylenglykol sein. Ein solches (Poly)alkylenglykol kann beispielsweise die Formel -O-[CHRₐCH₂O]_{b}-R_{b} aufweisen, wobei Rₐ H oder ein C₁₋₄-Alkylrest, R_{b}-H oder ein organischer Rest und b 1 bis 100 ist.

In den Verbindungen der Formel (I) ist X O, S, C(=O)O, OC(=O)O, C(=O)OC(=O)O, NRₓ, NRₓC(=O)O, NRₓC(=O)NRₓ oder OC(=O)NRₓ. In bevorzugten Ausführungsformen ist X O, OC(=O)O, NRₓ oder NRₓC(=O)O.

Jedes R und R' ist unabhängig ausgewählt aus H, C₁₋₂₀ Alkyl und C₂₋₂₀ Alkenyl, wobei insbesondere eines von R und R' H und das andere C₁₋₄ Alkyl oder beide H sind. Besonders bevorzugt ist R H und R' ist H oder -CH₃.

Jedes A, B und C ist unabhängig ausgewählt aus CR"R''', wobei R" und R''' unabhängig ausgewählt sind aus H, einer funktionellen Gruppe, wie beispielsweise -OH, -NH₂, -NO₂, -CN, -OCN, -SCN, -NCO, -SCH, -SH, -SO₃H oder -SO₂H, und einem organischen Rest. Insbesondere sind R" und R''' unabhängig H oder C₁₋₂₀ Alkyl. R" und R''' können aber auch gemeinsam oder zusammen mit dem Kohlenstoffatom an das sie gebunden sind einen organischen Rest, einschließlich zyklischer Reste, oder eine funktionelle Gruppe bilden. Beispiele für solche Reste sind =CH₂, =CH-Alkyl oder =C(Alkyl)₂, =O, =S, -(CH₂)ₐₐ- mit aa = 3 bis 5 bzw. Derivate davon, in denen eine oder mehrere Methylengruppen durch Heteroatome wie N, O oder S ersetzt sind. Zwei von R" und R''', die an benachbarte Kohlenstoffatome gebunden sind, können aber auch zusammen eine Bindung bilden. Dadurch wird zwischen den beiden benachbarten Kohlenstoffatome eine Doppelbindung ausgebildet (d.h. -C(R")=C(R")-).
- ------: steht für eine Einfach- oder Doppelbindung. Wenn es für eine Doppelbindung steht, trägt das Kohlenstoffatom, das an R₂ gebunden ist nur einen Substituenten R" oder R'''.

In den Verbindungen der Formel (I) ist m eine ganze Zahl von 1 bis 10, vorzugsweise 1 oder 2, besonders bevorzugt 1. D.h. die Verbindungen tragen vorzugsweise nur eine oder 2 Alkenylethergruppe(n).

n, p und o sind jeweils 0 oder eine ganze Zahl von 1 bis 10. Dabei erfüllen sie die Bedingung n+p+o=1 oder mehr, insbesondere 1 oder 2. Es ist besonders bevorzugt, dass n oder o 1 ist und die anderen 0 sind. Alternativ ist besonders bevorzugt, dass n oder o 2 ist und die anderen 0 sind. Es ist ferner bevorzugt, dass p 0 ist und eines von n und o 1 oder 2 ist und das andere 0 ist. Ebenfalls bevorzugt sind Ausführungsformen, in denen n und o 1 sind und p 0 ist.

Rₓ ist H oder

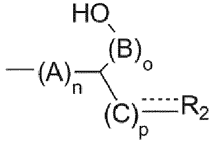

Damit das Alkenyletherpolyol mindestens zwei Hydroxylgruppen aufweist, erfüllt die Verbindung der

Formel (I) ferner die Bedingung, dass wenn X nicht NRₓ mit Rₓ = 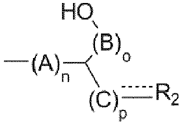 ist,

R₂ mindestens einen Substituenten aufweist, der ausgewählt ist aus -OH und

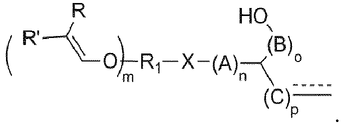

Die zweite Hydroxylgruppe der Verbindung der Formel (I) ist daher entweder als Substituent in dem organischen Rest R₂ enthalten oder X enthält einen weiteren Rest der Formel

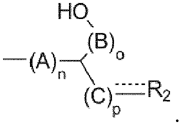

In verschiedenen Ausführungsformen des erfindungsgemäßen Herstellungsverfahren zur Darstellung eines Alkenyletherpolyols ist der Alkenylether, der mindestens eine Alkenylethergruppe und mindestens eine funktionelle Gruppe ausgewählt aus -OH, -COOH, -SH, -NH₂ und deren Derivaten enthält, ein Alkenylether der Formel (II).

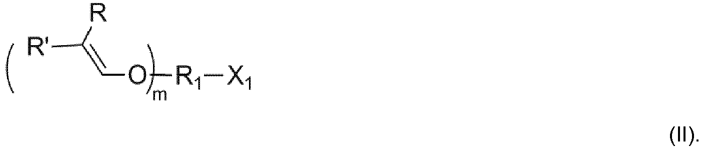

Ein solcher Alkenylether kann beispielsweise verwendet werden, um ein Alkenyletherpolyol der Formel (I) zu synthetisieren, indem er mit einem Epoxid oder einem cyclischen Carbonat umgesetzt wird.

In den Verbindungen der Formel (II) sind R₁, R, R' und m wie oben für Formel (I) definiert. Insbesondere sind die oben für die Verbindungen der Formel (I) beschriebenen bevorzugten Ausführungsformen von R₁, R, R' und m in gleicher Weise auf die Verbindungen der Formel (II) übertragbar.

In den Verbindungen der Formel (II) ist
X₁ eine funktionelle Gruppe ausgewählt aus -OH, -COOH, -SH, -NHR_{y} und deren Derivaten, und
R_{y} ist H oder ein organischer Rest, vorzugsweise H.

Die Derivate der funktionellen Gruppen -OH, -COOH, -SH, -NHR_{y} sind vorzugsweise die bereits oben im Zusammenhang mit der Definition des Begriffs beschriebenen ionischen Varianten, die sich durch Entfernen bzw. Bindung eines Protons ergeben, hierbei insbesondere die Alkoholate, Thiolate und Carboxylate, ganz besonders bevorzugt die Alkoholate.

Besonders bevorzugt ist X₁ -OH oder -O⁻ oder -NH₂.

Eine besonders bevorzugte Ausführungsform des erfindungsgemäßen Verfahrens ist weiterhin dadurch gekennzeichnet, dass in dem Alkenylether der Formel (II) m 1 ist, X₁ -OH oder -NH₂, vorzugsweise -OH, ist, R₁ ein zweiwertiger, linearer oder verzweigter C₁₋₁₀ Alkylrest (Alkylenylrest), insbesondere Ethylenyl, Propylenyl, Butylenyl, Pentylenyl oder Hexylenyl, ist, und eines von R und R' H und das andere H oder -CH₃ ist.

Bei den Alkenylethern, die im Rahmen der vorliegenden Erfindung eingesetzt werden können, insbesondere denen der Formel (II), kann es sich z. B. um Reaktionsprodukte von verschiedenen optional substituierten Alkanolen (Monoalkohole und Polyole) mit Acetylen handeln. Konkrete Beispiele schließen ein, ohne darauf beschränkt zu sein, 4-Hydroxybutylvinylether (HBVE); 2-Hydroxyethylvinylether; 2-(Hydroxyethoxy)ethylvinylether; Ethylenglykolmonovinylether; Propylenglykolmonovinylether, 1,4-Cyclohexandimethanolmonovinylether und 3-Aminopropylvinylether.

Eine weitere Ausführungsform des erfindungsgemäßen Verfahrens ist dadurch gekennzeichnet, dass das Epoxid, das mit dem Alkenylether umgesetzt wird, ein Epoxid der Formel (III) oder (IIIa) ist

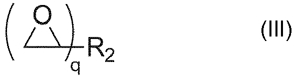

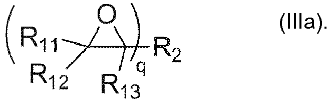

In Verbindungen der Formel (III) und (IIIa) ist R₂ wie oben für Formel (I) definiert.

R₁₁, R₁₂ und R₁₃ sind unabhängig voneinander H oder ein organischer Rest, optional mit mindestens einer -OH Gruppe, insbesondere ein lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom.

q ist eine ganze Zahl von 1 bis 10, vorzugsweise 1 oder 2.

Epoxyverbindungen, die in den erfindungsgemäßen Verfahren zur Herstellung von Alkenyletherpolyolen Anwendung finden können, sind demnach vorzugsweise lineare oder verzweigte, substituierte oder unsubstituierte Alkane mit einer Kohlenstoffatomanzahl von 1 bis 1000, vorzugsweise 1 bis 50 oder 1 bis 20, die mindestens eine Epoxygruppe tragen. Optional können diese Epoxyverbindungen zusätzlich noch eine oder mehrere Hydroxygruppen tragen, wodurch der Hydroxyl-Funktionalisierungsgrad des aus der Reaktion eines gegenüber Epoxiden reaktiven Alkenylethers, wie zuvor beschrieben, mit einem Epoxid entstandenen Alkenyletherpolyols hoch ist. Dadurch wiederum kann in späteren Polymerisierungsreaktionen die Quervernetzungsdichte des gewünschten Polymers kontrolliert und gesteuert werden.

Bei der Reaktion einer gegenüber Epoxiden reaktiven Alkenyletherverbindung (Alkenylether mit mindestens einer funktionellen Gruppe ausgewählt aus -OH, -COOH, -SH, -NH₂ und deren Derivaten) entsteht unter Ringöffnung des Epoxids ein Alkohol. Im Sinne der vorliegenden Erfindung wird aus der Reaktionen eines ersten Alkohols oder einer in diesem Kontext chemisch verwandten Verbindung (Amin, Thiol, Carbonsäure, etc.) mit einem Epoxid somit die alkoholische Gruppe im Zuge der Bindungsknüpfung "regeneriert".

In verschiedenen Ausführungsformen kann die Epoxyverbindung mehr als eine Epoxygruppe tragen. Dies ermöglicht die Umsetzung einer solchen Epoxyverbindung mit mehr als einer gegenüber Epoxiden reaktiven Alkenyletherverbindung, beispielsweise einem Aminoalkenylether oder Hydroxyalkenylether.

In besonders bevorzugten Ausführungsformen ist das Epoxid ein Epoxid der Formel (III), wobei q 1 oder 2 ist, und wenn q 2 ist, R₂ -CH₂-O-C₁₋₁₀-Alkylenyl-O-CH₂- ist, und wenn q 1 ist, R₂ -CH₂-O-C₁₋₁₀-Alkyl ist.

Beispiele für Epoxyverbindugen, die in den erfindungsgemäßen Herstellungsverfahren zum Einsatz kommen können, sind insbesonders Glycidylether, wie z.B., ohne Einschränkung, 1,4-Butandioldiglycidylether (BDDGE); Neopentyldiglycidylether; Cyclohexandimethanoldiglycidylether; Hexandioldiglycidylether, Polypropylenglycoldiglycidylether; Polyetylenglycoldiglycidylether; Mono-, Di- oder Triglycidylether von ethyoxliertem oder propoxyliertem Glycerin und Isopropylglycidylether (IPGE).

In verschiedenen Ausführungsformen, ist das Alkenyletherpolyol der Formel (I) durch Umsetzen eines Alkenylethers der Formel (II) mit einem Epoxid der Formel (III) oder (IIIa) darstellbar.

Anstelle eines Epoxids kann es sich bei den Verbindungen, die mit den gegenüber Epoxiden reaktiven Verbindungen (Alkenyletherverbindungen) umgesetzt werden, auch um cyclische Carbonate oder deren Derivate handeln. Im Sinne der vorliegenden Erfindung unterliegen cyclische Carbonatverbindungen einer ähnlich den Epoxiden gearteten Reaktivität gegenüber den als Reaktionspartner dienenden Verbindungen, die sowohl Epoxide als auch cyclische Carbonatverbindungen unter Ringöffnung und "Regeneration" einer alkoholischen funktionellen Gruppe nucleophil am, im Falle eines Epoxids, Methylen des Epoxidrings, oder, im Falle eines cyclischen Carbonats, Carbonyl-Kohlenstoffatom addieren, wodurch, in Abhängigkeit des reaktiven, nucleophilen Restes, eine O-C-, N-C, S-C, oder O-/N-/S-C(=O)O-Bindung geknüpft wird.

Die cyclischen Carbonate, welche im erfindungsgemäßen Verfahren mit einem Alkenylether, insbesondere einem Alkenylether der Formel (II), umgesetzt werden können, sind in bevorzugten Ausführungsformen Ethylencarbonate der Formel (IV) oder (IVa)

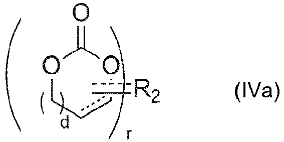

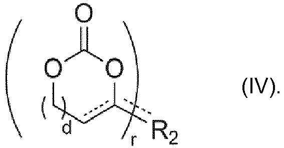

In Verbindungen der Formel (IV) und (IVa) ist R₂ wie für Formel (I), (III) und (IIIa) definiert. Insbesondere ist R₂ ein C₁₋₁₀ Hydroxyalkyl. In weiteren Ausführungsformen kann R₂ =CH₂ sein.
------ ist eine Einfach- oder Doppelbindung, vorzugsweise eine Einfachbindung. Es ist selbstverständlich, dass wenn der Ring eine Doppelbindung enthält, R₂ nicht über eine exo-Doppelbindung sondern über eine Einfachbindung gebunden ist und umgekehrt.

d ist 0, 1, 2, 3, 4 oder 5, vorzugsweise 0 oder 1, besonders bevorzugt 0, und r ist eine ganze Zahl von 1 bis 10, vorzugsweise 1 oder 2, ganz besonders bevorzugt 1.

Wenn d 1 ist, d.h. das Cyclocarbonat ein 1,3-Dioxan-2-on ist, kann R₂ in der 4- oder 5-Position sein, ist aber vorzugsweise in der 5-Position.

Beispielhafte cyclische Carbonate schließen ein, ohne darauf beschränkt zu sein, 1,3-Dioxolan-2-on, 4,5-Dehydro-1,3-Dioxolan-2-on, 4-Methylen-1,3-Dioxolan-2-on, und 1,3-Dioxan-2-on, die in der 4-bzw. 5-Position mit R₂ substituiert sind.

In verschiedenen Ausführungsformen der Erfindung werden cyclische Carbonat eingesetzt, die Derivate der Carbonate der Formeln (IV) und (IVa) sind. Beispielhafte Derivate schließen solche ein, die an den Ring-Methylengruppen, die nicht den R₂ Rest tragen, substituiert sind, beispielsweise mit organischen Resten, insbesondere linearen oder verzweigten, substituierten oder unsubstituierten Alkyl- oder Alkenylresten mit bis zu 20 Kohlenstoffatomen, insbesondere =CH₂ und -CH=CH₂, oder linearen oder verzweigten, substituierten oder unsubstituierten Heteroalkyl- oder Heteroalkenylresten mit bis zu 20 Kohlenstoffatomen und mindestens einem Sauerstoff- oder Stickstoffatom, oder funktionellen Gruppen, wie beispielsweise -OH oder -COOH. Beispiele für solche Derivate schließen beispielsweise 4-Methylen-1,3-Dioxolan-2-on, das an der 5-Position den R₂ Rest trägt, ein.

In verschiedenen Ausführungsformen in denen der R₂ Rest über eine Einfachbindung gebunden ist, kann das Ring-Kohlenstoffatom, dass den R₂ Rest trägt mit einem weiteren Substituenten, der wie die oben genannten Substituenten für die anderen Ring-Methylengruppe definiert ist, substituiert sein.

Weitere Derivate sind solche in denen eines oder beide der Ring-Sauerstoffatome durch Schwefelatome ersetzt sind sowie solche in denen alternativ oder zusätzlich das Carbonyl-Sauerstoffatom durch ein Schwefelatom ersetzt ist. Ein besonders bevorzugtes Derivat ist das 1,3-Oxathiolan-2-thion.

In verschiedenen Ausführungsformen ist das cyclische Carbonat 4-Methylen-1,3-Dioxolan-2-on, das an der 5-Position den R₂ Rest trägt. Wenn ein solches cyclisches Carbonat mit einem Alkylether umgesetzt wird, der als reaktive Gruppe eine Aminogruppe trägt, kann sich eine Verbindung der Formel (la) bilden:

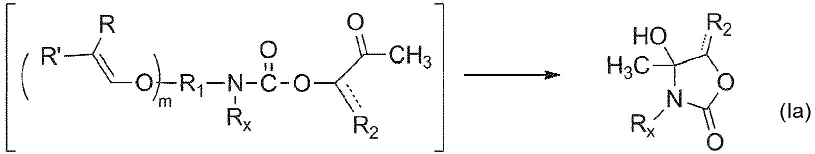

In dieser Verbindung sind m, R₁, R, R', R₂ und Rₓ wie oben für die Verbindungen der Formel (I)-(IV) definiert.

Bei der Umsetzung der vorstehend beschriebenen Cyclocarbonate und deren Derivate der Formeln (IV) und (IVa) mit einer Verbindung der Formel (II), ist in verschiedenen Ausführungsformen in den Verbindungen der Formel (II) (i) X₁ -NH₂ oder ein Derivat davon, und q oder r ist 1; oder (ii) X₁ ist -OH oder ein Derivat davon, und q oder r ist 2.

In weiteren Ausführungsformen ist das Alkenyletherpolyol durch Umsetzen der in Route B) aufgeführten Verbindungen darstellbar. Dabei wird das Alkenyletherpolyol hergestellt durch Umsetzen eines Alkenylethers, enthaltend mindestens eine Alkenylethergruppe und mindestens eine funktionelle Gruppe ausgewählt aus (i) Epoxidgruppen und (ii) cyclischen Carbonatgruppen oder Derivaten davon, mit einem Alkohol, Thiol, einer Carbonsäure oder einem Amin oder Derivaten der vorgenannten.

In verschiedenen Ausführungsformen dieses Verfahrens ist das Alkenyletherpolyol ein Alkenyletherpolyol der Formel (V)

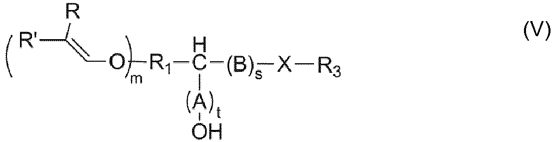

In den Verbindungen der Formel (V) ist R₁ wie oben für die Verbindungen der Formel (I) definiert. R₃ ist ein (optional 2- oder mehrwertiges) lineares oder verzweigtes, substituiertes oder unsubstituiertes Alkyl mit 1 bis 50, vorzugsweise 1 bis 20 Kohlenstoffatomen oder ein (optional 2- oder mehrwertiges) lineares oder verzweigtes, substituiertes oder unsubstituiertes Heteroalkyl mit 1 bis 50, vorzugsweise 1 bis 20 Kohlenstoffatomen und mindestens einem Sauerstoff- oder Stickstoffatom, jeweils optional mit mindestens einer -OH Gruppe. R₃ kann aber auch ein hochmolekularer Rest, wie beispielweise ein Polyalkylenglykol sein. Ein solches (Poly)alkylenglykol kann beispielsweise die Formel -O-[CHRₐCH₂O]_{b}-R_{b} aufweisen, wobei Rₐ H oder ein C₁₋₄-Alkylrest, R_{b}-H oder ein organischer Rest oder 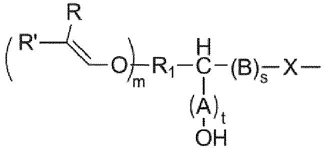 ist und b 1 bis 100 ist.

In den Verbindungen der Formel (V) ist X O, S, OC(=O), OC(=O)O, OC(=O)OC(=O), NR_{z}, NR_{z}C(=O)O, NR_{z}C(=O)NR_{z} oder OC(=O)NR_{z}. In bevorzugten Ausführungsformen ist X O, OC(=O)O, NR_{z} oder OC(=O)NR_{z}.

Jedes R und R' ist unabhängig ausgewählt aus H, C₁₋₂₀ Alkyl und C₂₋₂₀ Alkenyl, wobei insbesondere eines von R und R' H und das andere C₁₋₄ Alkyl oder beide H sind. Besonders bevorzugt ist R H und R' ist H oder -CH₃.

Jedes A und B ist unabhängig ausgewählt aus CR"R''', wobei R" und R''' unabhängig ausgewählt sind aus H, einer funktionellen Gruppe, wie beispielsweise -OH, -NH₂, -NO₂, -CN, -OCN, -SCN, -NCO, - SCH, -SH, -SO₃H oder -SO₂H, und einem organischen Rest. Insbesondere sind R" und R''' unabhängig H oder C₁₋₂₀ Alkyl. R" und R''' können aber auch gemeinsam oder zusammen mit dem Kohlenstoffatom an das sie gebunden sind einen organischen Rest, einschließlich zyklischer Reste, oder eine funktionelle Gruppe bilden. Beispiele für solche Reste sind =CH₂, =CH-Alkyl oder =C(Alkyl)₂, =O, =S, -(CH₂)ₐₐ- mit aa = 3 bis 5 bzw. Derivate davon, in denen eine oder mehrere Methylengruppen durch Heteroatome wie N, O oder S ersetzt sind. Zwei von R" und R''', die an benachbarte Kohlenstoffatome gebunden sind, können aber auch zusammen eine Bindung bilden. Dadurch wird zwischen den beiden benachbarten Kohlenstoffatome eine Doppelbindung ausgebildet (d.h. -C(R")=C(R")-).

In den Verbindungen der Formel (V) ist m eine ganze Zahl von 1 bis 10, vorzugsweise 1 oder 2, besonders bevorzugt 1. D.h. die Verbindungen tragen vorzugsweise nur eine oder 2 Alkenylethergruppe(n).

s und t sind jeweils 0 oder eine ganze Zahl von 1 bis 10. Dabei erfüllen sie die Bedingung s+t=1 oder mehr, insbesondere 1 oder 2. Es ist besonders bevorzugt, dass s oder t 1 ist und die andere 0 ist.

R_{z} ist H oder

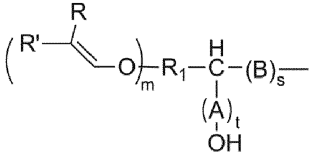

Damit das Alkyletherpolyol der Formel (V) die Bedingung erfüllt, dass es mindestens zwei Hydroxylgruppen trägt, ist wenn X nicht NR_{z} mit R_{z} = 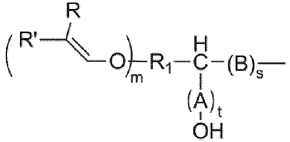 ist,
R₃ mit mindestens einem Substituenten substituiert, der ausgewählt ist aus -OH und

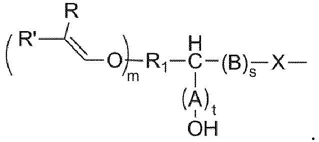

In weiteren bevorzugten Ausführungsformen ist das erfindungsgemäße Verfahren dadurch gekennzeichnet, dass der Alkenylether, der mindestens eine Alkenylethergruppe und mindestens eine funktionelle Gruppe ausgewählt aus (i) Epoxidgruppen und (ii) cyclischen Carbonatgruppen oder Derivaten davon enthält, ein Alkenylether der Formel (VI) oder (VII) ist

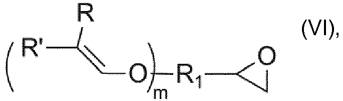

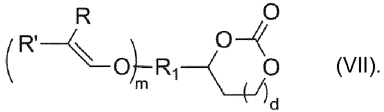

In den Verbindungen der Formel (VI) oder (VII) sind R₁, R, R' und m wie oben für die Verbindungen der Formeln (I) und (II) definiert.

d ist wie oben für die Formeln (IV) und (IVa) definiert, d.h. d ist 0, 1, 2, 3, 4 oder 5, vorzugsweise 0 oder 1, besonders bevorzugt 0.

In besonders bevorzugten Ausführungsformen ist R₁ in den Alkenylethern der Formel (VI) oder (VII) -C₁₋₁₀-Alkylenyl-O-CH₂-.

Die Epoxygruppen tragenden Alkenylether der Formel (VI) können am Epoxyrest substituiert sein, d.h. die Methylengruppen des Oxiranrings können, wie in Formel (IIIa) gezeigt, mit R₁₁-R₁₃ substituiert sein.

In verschiedenen Ausführungsformen sind die Alkenylether der Formel (VII) am Cyclocarbonatring substituiert oder der Cyclocarbonatring ist durch ein entsprechendes Derivat ersetzt. Geeignete substituierte Cyclocarbonate sowie Derivate davon sind solche, die oben im Zusammenhang mit Formel (IV) und (IVa) beschrieben wurden. Insbesondere ist der Cyclovarbonatrest vorzugsweise ein 1,3-Dioxolan-2-on- oder 1,3-Dioxan-2-on-Rest, der ggf. substituiert sein kann, beispielsweise mit einer Methylengruppe.

Geeignete Verbindungen der Formel (VI) schließen ein, ohne darauf beschränkt zu sein, Vinylglycidylether und 4-Glycidylbutylvinylether (GBVE), wobei letzterer durch Umsetzen von 4-Hydroxybutylvinylether mit Epichlorhydrin darstellbar ist.

Geeignete Verbindungen der Formel (VII) schließen ein, ohne darauf beschränkt zu sein, 4-(Ethenyloxymethyl)-1,3-Dioxolan-2-on, das beispielsweise durch Umesterung von Glycerincarbonat mit Ethylvinylether.

In verschiedenen Ausführungsformen wird der Alkenylether, der mindestens eine Alkenylethergruppe und mindestens eine funktionelle Gruppe ausgewählt aus (i) Epoxidgruppen und (ii) cyclischen Carbonatgruppen oder Derivaten davon enthält, insbesondere einer der Formel (VI) oder (VII) mit einem Alkohol umgesetzt. Bei dem Alkohol kann es sich um ein Diol oder Polyol oder ein entsprechendes Alkoholat handeln. Insbesondere kann der Alkohol ein Polyalkylenglykol der Formel HO-[CHRₐCH₂O]_{b}-H sein, wobei Rₐ H oder ein C₁₋₄-Alkylrest und b 1 bis 100, insbesondere 1 bis 10, ist.

Route B) stellt somit eine alternative Ausführungsform der vorliegenden Erfindung dar, in der die Epoxid- oder die cyclischen Carbonatverbindungen (beispielsweise Ethylencarbonat- oder Trimethylencarbonatverbindungen) mindestens eine oder mehrere Alkenylethergruppen aufweisen. Die Umsetzung dieser Epoxid- oder cyclischen Carbonatverbindungen mit gegenüber Epoxiden oder im Kontext dieser Erfindung chemisch ähnlich reagierenden Verbindungen (cyclische Carbonate) reaktiven Verbindungen, insbesondere solchen, die -OH, -COOH, -SH, -NH₂ und ähnliche Gruppen oder deren Derivate tragen - beispielsweise entsprechend funktionalisierte, vorzugsweise mehrfach entsprechend funktionalisierte lineare oder verzweigte, gesättigte oder teilweise ungesättigte, zusätzlich substituierte oder unsubstituierte, zyklische oder lineare (Hetero)Alkyle und (Hetero)Aryle - ergibt die gewünschten Alkenyletherpolyole.

Beispiele für Verbindungen, die mindestens eine der Gruppen -OH, -COOH, -SH, -NH₂ und deren derivatisierte Formen aber keine Alkenylethergruppen aufweisen, sind beispielsweise, ohne Einschränkung, Glykole, Polyglykole, Glycin, Glycerol, Hexamethylendiamin, 1,4-Butandiol und 1,6-Hexandiol.

Die in mittels der beschriebenen Verfahren herstellbaren oder erhältlichen Alkenyletherpolyole sind ebenfalls Gegenstand der Erfindung. In verschiedenen Ausführungsformen handelt es sich dabei um Verbindungen der Formeln (I), (la) und (V), wie oben definiert.

In verschiedenen Ausführungsformen der Alkenyletherpolyole der Formel (I) ist:
(1) m = 1; R und R' sind H oder R ist H und R' ist Methyl; R₁ ist C₁₋₁₀ Alkylenyl, insbesondere C₁₋₆ Alkylenyl, X ist O, A und B sind CH₂, n und o sind 1 oder 0 und p ist 0, wobei n+o=1, und R₂ ist ein organischer Rest wie oben definiert, der mit -OH substituiert ist oder einen weiteren Rest der Formel 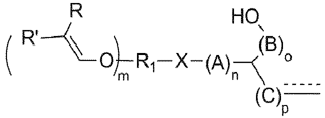 trägt wobei R₁, m, R, R', A, B, C, n, o und p wie vorstehend definiert sind; oder
(2) m = 1; R und R' sind H oder R ist H und R' ist Methyl; R₁ ist C₁₋₁₀ Alkylenyl, insbesondere C₁₋₆ Alkylenyl, X ist NRₓ, A und B sind CH₂, n und o sind 1 oder 0 und p ist 0, wobei n+o=1, Rₓ ist H oder 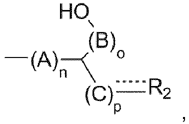 wobei A, B, C, n, o und p wie vorstehend definiert sind; und R₂ ist ein organischer Rest wie oben definiert, der wenn Rₓ H ist mit -OH substituiert ist oder einen weiteren Rest der Formel 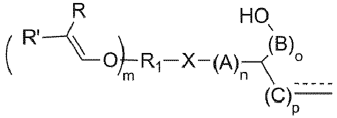 trägt wobei R₁, m, R, R', A, B, C, n, o und p wie vorstehend definiert sind; oder
(3) m = 1; R und R' sind H oder R ist H und R' ist Methyl; R₁ ist C₁₋₁₀ Alkylenyl, insbesondere C₁₋₆ Alkylenyl, X ist OC(=O)O, A und B sind CH₂, n und o sind 1 oder 0 und p ist 0, wobei n+o=1, und R₂ ist ein organischer Rest wie oben definiert, der mit -OH substituiert ist oder einen weiteren Rest der Formel 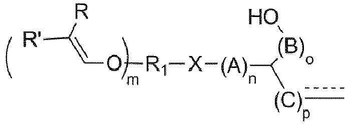 trägt wobei R₁, m, R, R', A, B, C, n, o und p wie vorstehend definiert sind; oder
(4) m = 1; R und R' sind H oder R ist H und R' ist Methyl; R₁ ist C₁₋₁₀ Alkylenyl, insbesondere C₁₋₆ Alkylenyl, X ist NRₓC(=O)O, A und B sind CH₂, n und o sind 1 oder 0 und p ist 0, wobei n+o=1, Rₓ ist H oder 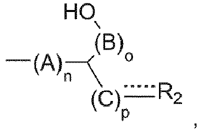 wobei A, B, C, n, o und p wie vorstehend definiert sind; und R₂ ist ein organischer Rest wie oben definiert, der wenn Rₓ H ist mit -OH substituiert ist oder einen weiteren Rest der Formel 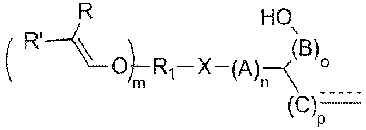 trägt wobei R₁, m, R, R', A, B, C, n, o und p wie vorstehend definiert sind.

In den vorstehend genannten Ausführungsformen ist R₂ vorzugsweise über eine Einfachbindung gebunden und kann beispielsweise ein Heteroalkylrest, insbesondere ein Alkyletherrest mit 2 bis 10 Kohlenstoffatomen sein. Geeignet sind beispielsweise Reste der Formel -CH₂-O-(CH₂)₄-O-CH₂- (für den Fall, dass R₂ zwei Alkenyletherreste der obigen Formel trägt) oder -CH₂-O-CH(CH₃)₂.

In verschiedenen Ausführungsformen der Alkenyletherpolyole der Formel (V) ist:
(1) m = 1; R und R' sind H oder R ist H und R' ist Methyl; R₁ ist -(CH₂)₁₋₁₀-O-CH₂-, insbesondere -(CH₂)₁₋₆-O-CH₂-, X ist O, A und B sind CH₂, s und t sind 1 oder 0, wobei s+t=1, und R₃ ist ein organischer Rest wie oben definiert, der mit -OH substituiert ist oder einen weiteren Rest der Formel 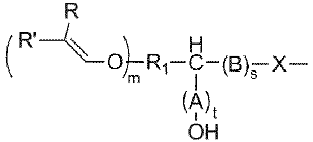 trägt wobei R₁, m, R, R', A, B, s und t wie vorstehend definiert sind; oder
(2) m = 1; R und R' sind H oder R ist H und R' ist Methyl; R₁ ist -(CH₂)₁₋₁₀-O-CH₂-, insbesondere -(CH₂)₁₋₆-O-CH₂-, X ist NR_{z}, A und B sind CH₂, s und t sind 1 oder 0, wobei s+t=1, R_{z} ist H oder 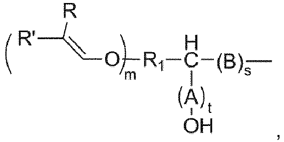 wobei A, B, m, s und t wie vorstehend definiert sind; und R₃ ist ein organischer Rest wie oben definiert, der wenn R_{z} H ist mit -OH substituiert ist oder einen weiteren Rest der Formel 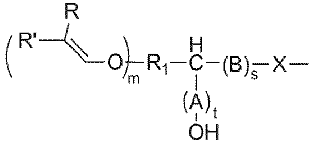 trägt wobei R₁, m, R, R', A, B, s und t wie vorstehend definiert sind; oder
(3) m = 1; R und R' sind H oder R ist H und R' ist Methyl; R₁ ist -(CH₂)₁₋₁₀-O-CH₂-, insbesondere -(CH₂)₁₋₆-O-CH₂-, X ist OC(=O)O, A und B sind CH₂, s und t sind 1 oder 0, wobei s+t=1, und R₃ ist ein organischer Rest wie oben definiert, der mit -OH substituiert ist oder einen weiteren Rest der Formel 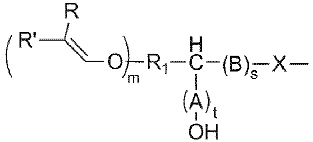 trägt wobei R₁, m, R, R', A, B, s und t wie vorstehend definiert sind; oder
(4) m = 1; R und R' sind H oder R ist H und R' ist Methyl; R₁ ist -(CH₂)₁₋₁₀-O-CH₂-, insbesondere -(CH₂)₁₋₆-O-CH₂-, X ist OC(=O)NR_{z}, A und B sind CH₂, s und t sind 1 oder 0, wobei s+t=1, R_{z} ist H oder 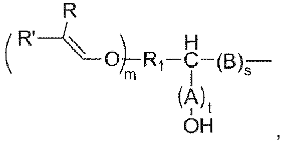 wobei A, B, m, s und t wie vorstehend definiert sind; und R₃ ist ein organischer Rest wie oben definiert, der wenn R_{z} H ist mit -OH substituiert ist oder einen weiteren Rest der Formel 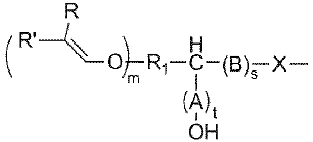 trägt wobei R₁, m, R, R', A, B, s und t wie vorstehend definiert sind.

In den vorstehend genannten Ausführungsformen der Verbindungen der Formel (V) ist R₃ beispielsweise ein Heteroalkylrest, insbesondere ein (Poly)Alkylenglykol, wie insbesondere Polypropylenglykol, oder ein C₁₋₁₀ Alkyl- oder Alkylenylrest.

Die Durchführung der einzelnen Stufen des erfindungsgemäßen Verfahrens zur Herstellung der Verbindungen (I) oder (V) kann nach den für solche Reaktionen üblichen Methoden erfolgen. Dazu werden die Reaktionspartner, ggf. nach Aktivierung (beispielsweise Herstellung von Alkoholaten durch Umsetzen mit Natrium, miteinander in Kontakt gebracht und ggf. unter Schutzgasatmosphäre und Kontrolle der Temperatur umgesetzt.

Die Alkenyletherpolyole, die mittels des erfindungsgemäßen Verfahrens hergestellt werden können, sind geeignete Vorstufen für die Synthese von UV-vernetzbaren Oligomeren oder Polymeren mittels Polyadditions- oder Polykondensationsreaktionen, insbesondere zur Synthese von UV-härtbaren Polyestern, Polyethern, Polyurethanen und Polyharnstoffen, besonders bevorzugt UV-härtbaren Polyurethanen. Deren Verwendung in derartigen Synthesen bzw. Verfahren zur Herstellung solcher Polymere unter Verwendung der beschriebenen Alkenyletherpolyole sind weiterere Aspekte der vorliegenden Erfindung. Insbesondere sind die erfindungsgemäß hergestellten Alkenyletherpolyole zur Herstellung von UV-härtbaren Polyurethanpolymeren geeignet, die durch Umsetzen mindestens eines Alkenyletherpolyols im Sinne der vorliegenden Erfindung mit einem Polyisocyanat erhältlich sind.

Verfahren zur Herstellung von UV-härtbaren Polyurethanpolymeren sind im Stand der Technik bekannt und werden beispielsweise in Beispiel 6 der vorliegenden Erfindung beschrieben.

Im Folgenden werden Verfahren zur Herstellung von UV-härtbaren Polyurethanpolymeren beispielhaft beschrieben. Es ist selbstverständlich, dass die Erfindung nicht auf solche Ausführungsformen beschränkt ist, sondern diese vielmehr dazu dienen, diesen Aspekt der Erfindung weiter zu veranschaulichen.

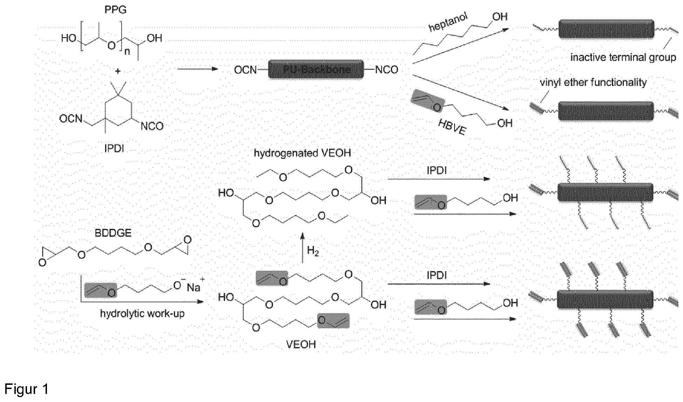

Unter Verwendung der hierin beschriebenen Alkenyletherpolyole, insbesondere der beschriebenen Vinyletherpolyole, können hochgradig Vinylether-funktionalisierte Polyurethane (VEPUs) hergestellt werden, indem über die neuartigen Polyole aktive Seitenketten in das Polymerrückgrat eingebaut werden. In einem konkreten Beispiel kann beispielweise 4-Hydroxybutylvinylether (HVBE) deprotoniert und mit 1,4-Butandioldiglycidylether (BDDGE) umgesetzt werden, um das Vinylether-funktionalisierte Diol (VEOH) zu ergeben. Bei der Ringöffnung durch den nukleophilen Angriff des Alkoholats erzeugt jede Epoxygruppe eine neue Hydroxylgruppe. Das entstandene VEOH wird dann mit Isophorondiisocyanat umgesetzt, um das Seitenketten-Vinylether-funktionalisierte PolyurethanPräpolymer zu ergeben (sc-VEPU). Die terminalen Isocyanatgruppen können dann im Folgenden mit HBVE umgesetzt werden, um auch die Endgruppen mit Vinylethergruppen zu funktionalisieren. Eine genauere Beschreibung der Synthese findet sich in den Beispielen 6 und 7. Der Reaktionsverlauf ist schematisch in Figur 1 gezeigt.

Die Erfindung betrifft schließlich auch die mittels der hierin beschriebenen Alkenyletherpolyole herstellbaren Polymere, insbesondere die UV-härtbaren Polyurethane. Diese Polyurethane können auch in Form von wasserbasierten Dispersionen (PUD) eingesetzt werden.

Die hierin beschriebenen Alkenylether-funktionalisierten Polyole erlauben die Synthese von neuartigen Polyurethanen (PU), die mittels eines kationischen Polymerisationsmechanismus mit UV-Strahlung vernetzt (gehärtet) werden können. Eine derartige Aushärtung steht im Gegensatz zu bekannten Verfahren zur Aushärtung von UV-härtbaren Polyurethanen, die auf radikalischer Polymerisation basieren (wie z.B. acrylat-funktionalisierte Polyurethane). Derartige radikalische Mechanismen haben den Nachteil, dass sie gegenüber Sauerstoff empfindlich sind, d.h. die Anwesenheit von Sauerstoff die Reaktion inhibieren kann, was insbesondere für Dünnschichtanwendungen und Beschichtungen ein schwerwiegender Nachteil ist.

Des Weiteren erlaubt die kationische UV Härtung die Bereitstellung von "dark cure" Eigenschaften, d.h. nach einem kurzen Strahlungspuls, der für die Aktivierung notwendig ist und die Polymerisation oder Härtung startet, verläuft die Reaktion ohne weitere Bestrahlung, d.h. unabhängig von der UV-Strahlungsquelle. Die Reaktion kann also nach dem Start im Dunkeln (= dark cure) oder auf einer Fertigungsstraße weiterlaufen, was insbesondere für Klebstoffanwendungen, z.B. in voll automatisierten Prozessen, ein wichtiger Vorteil ist.

Ein Vorteil der mit den hierin beschriebenen Alkenyletherpolyolen hergestellten Polymere liegt somit in der chemischen Reaktionsfähigkeit der Alkenyletherfunktionalisierung. Neben der bereits beschriebenen Möglichkeit zur UV Härtung sind auch andere Reaktionen möglich, wie z.B. die Addition von Thiolen als weitere Vernetzungsmöglichkeit.

Ein weiterer Vorteil ist, dass die beschriebenen Alkenyletherpolyole die kontrollierte Synthese von Polymeren mit einem festgelegten Anteil an UV-härtbaren Alkenylethergruppen erlauben. Bekannte UV-härtbare Polymere enthalten oftmals terminale Vinylethergruppen und werden über Verkappung der Endgruppen mit Vinylethern hergestellt. Solche Polymere können aufgrund der beschränkten Anzahl von Endgruppen, typischerweise nur 2, keine Polymersysteme mit hohen Quervernetzungsdichten oder deutlichen Veränderungen der mechanischen Moduli nach der Härtung liefern. Die hierin beschriebenen Alkenyletherpolyole stellen somit eine Alternative zu den bisher eingesetzten monofunktionellen, kommerziell erhältlichen Vinylether-funktionalisierten Alkoholen, wie z.B. 4-Hydroxybutylvinylether, Cyclohexyldimethanolmonovinylether oder 2-Hydroxyethylvinylether, die als Endgruppenverkappungsmittel verwendet werden können, dar. Aufgrund der höheren Konzentration und der daraus folgenden engeren Vernetzungsdichte, ist die Härtung im Vergleich zu terminal funktionalisierten Vinylether Polyurethanen mit einer deutlicheren Änderung der mechanischen Eigenschaften verbunden. So können klebefreie Filme mit um Größenordnungen höheren Schermodulen erhalten werden.

Die Alkenyletherpolyole, insbesondere die Vinyletherpolyole, die hierin beschrieben werden, können somit zusätzlich oder als Alternative zu bekannten Polyolen für die Synthese von Polymeren, insbesondere Polyurethanen eingesetzt werden. Bekannte Polyole, die für die PU Synthese verwendet werden, schließen beispielsweise Polyether- und Polyesterpolyole ein, sind aber nicht darauf beschränkt. Für die Polyurethansynthese werden die Polyole oder Gemische von Polyolen, die die beschriebenen Alkenyletherpolyole enthalten, mit Polyisocyanaten, typischerweise im molaren Überschuss, umgesetzt. Die Reaktion erfolgt hierbei unter an sich bekannten Bedingungen, d.h. bei erhöhter Temperatur und ggf. in Gegenwart eines Katalysators. Abhängig von der Menge an eingesetztem Alkenyletherpolyol weisen die erhaltenen Polyurethan(prä)polymere die gewünschte Dichte an quervernetzbaren Alkenylethergruppen auf. Beispiele für derart synthesierte Polyurethane wurden bereit oben im Zusammenhang mit den beanspruchten Verfahren beschrieben.

Alle hierin im Zusammenhang mit den erfindungsgemäßen Verfahren zur Herstellung der Alkenyletherpolyole offenbarten Ausführungsformen sind ebenso auf die beschriebenen Alkenyletherpolyole als solche, sowie deren Verwendung, Verfahren zu deren Verwendung, die damit synthetisierten Polymere und deren Verwendungen übertragbar und umgekehrt.

Die Erfindung wird im Folgenden anhand von Beispielen weiter veranschaulicht, wobei diese nicht als Einschränkung verstanden werden sollen.

### Beispiele

### Verwendete Materialien:

4-Hydroxybutylvinylether (HBVE) (BASF) und 3-Aminopropylvinylether (APVE) (BASF) wurden über Molekularsieb 4 Å gelagert.

Natrium (Merck) wurde in trockenem Diethylether gewaschen und in Stücke geschnitten. 1,4-Butandioldiglycidylether (BDDGE, Sigma-Aldrich, 95 %), 2,3-Epoxypropanol (Glydidol, Glycid; Evonik), Isopropylglycidylether (IPGE, Raschig), Epichlorhydrin (Solvay, 99,8 %), Isophorondiisocyanat (IPDI) (Merck, 99%), Polypropylenglycol (PPG) (Dow Chemical, Voranol 2000 L, 2000 g/mol), 1-Heptanol (Acros Organics, 98%), Dimethylzinndineodecanoat (Momentive, Fomrez Katalysator UL-28), 4,4'-Dimethyldiphenyliodoniumhexafluorophosphat (Omnicat 440, IGM 98%), Hexamethylendiamin (99%, Merck), Tetrabutylammoniumbromid (TBAB, 99%, Acros Organics) und Natriumhydroxid (Riedel-de-Häen, 99 %) wurden wie erhalten verwendet.

### Beispiel 1: Synthese eines Vinyletherpolyols (VEOH)

139,51 g (1,2 mol) HBVE wurden in einem 250 ml Rundkolben vorgelegt. Ein Tropftrichter mit Druckausgleich wurde angeschlossen und darin 24,78 g (0,12 mol) BDDGE vorgelegt. Die gesamte Apparatur wurde im Vakuum getrocknet und mit Stickstoff geflutet. 7,00 g (0,3 mol) Natrium wurden zugefügt. Nachdem das Natrium vollständig aufgelöst war, wurde BDDGE langsam zugegeben. Die Temperatur wurde so kontrolliert, dass sie 50 °C nicht überschritt. Nach vollständiger Zugabe von BDDGE wurde bei 50 °C über einen Zeitraum von 30 min gerührt. 50 ml Wasser wurden zugegeben, um das verbliebene Alkoholat zu hydrolysieren. Das Produkt wurde mehrere Male mit gesättigter Natriumchlorid-Lösung und Wasser gewaschen und im Vakuum aufkonzentriert, um gegebenenfalls Edukt- und Wasserrückstände zu entfernen. Ausbeute: 76 %. ¹H-NMR (CDCl₃), xy MHz): δ (pp) = 1,6-1,8 (12 H, mid-CH₂ Butyl), 2,69 (2 H, OH, H/D austauschbar), 3,4-3,55 (16 H, CH₂-O-CH₂), 3,70 (4 H, CH₂-O-vinyl), 3,94 (2 H, CH-O), 3,98 (1 H, CH₂=CH-O trans), 4,17 (1 H, CH₂=CH-O cis), 6,46 (1 H, CH₂=CH-O gemi).

### Beispiel 2:

50,58 g (0,5 mol) APVE und 139,44 g (81,2 mol) IPGE wurden in einem 250 ml Rundkolben vorgelegt und zum Rückfluss erhitzt. Die voranschreitende exotherme Reaktion wurde so kontrolliert, dass eine Temperatur von 175 °C nicht überschritten wurde. Die Reaktion wurde auf Raumtemperatur abgekühlt und, nachdem IR-Spektroskopie den Umsatz der gewünschten Menge an Epoxid anzeigte, wurden 20 ml Natriumhydroxid (1 mol/l) zugefügt und die Emulsion auf 100 °C über einen Zeitraum von 30 min erhitzt, um verbliebene Epoxidrückstände zu hydrolysieren. Die organische Phase wurde mehrere Male mit Wasser gewaschen und unter vermindertem Druck getrocknet. Ausbeute: 54 %. ¹H-NMR (CDCl₃, xy MHz): δ (pp) = 1,15 (12 H, CH₃), 1,82 (2 H, mid-CH₂ Propyl), 2,45-2,80 (6 H, CH₂-N), 3,05-3,30 (2 H, OH), 3,40 (4 H, CH₂-O-isopropyl), 3,59 (2 H, CH Isopropyl), 3,73 (2 H, CH₂-O-vinyl), 3,81 (2 H, CH-OH), 3,99 (1 H, CH₂=CH-O trans), 4,18 (1 H, CH₂=CH-O cis), 6,45 (1 H, CH₂=CH-O gemi).

### Beispiel 3:

58,08 g (0,5 mol) HBVE wurden in einem 250 ml Rundkolben vorgelegt. Ein Tropftrichter mit Druckausgleich wurde angeschlossen und darin 7,41 g (0,13 mol) Glycidol vorgelegt. Die Apparatur wurde im Vakuum getrocknet und mit Stickstoff geflutet. 3,00 g Natrium (0,13 mol) wurden zugefügt. Nachdem das Natrium vollständig aufgelöst war, wurde Glycidol langsam zugegeben. Die Temperatur wurde so kontrolliert, dass sie 50 °C nicht überschritt. Die Mischung wurde über einen Zeitraum von 30 min bei 50 °C gerührt, nachdem Glycidol vollständig zugegeben worden war. 50 ml Wasser wurden zugegeben um verbliebene Alkoholate zu hydrolysieren. Das Produkt wurde mehrere Male mit gesättigter Natriumchlorid-Lösung gewaschen und im Vakuum aufkonzentriert um eventuell verbliebene Edukt- und Wasserrückstände zu entfernen. Ausbeute: 77 %. ¹H-NMR (CDCl₃, xy MHz): δ (pp) = 1,6-1,8 (4 H, mid-CH₂ Butyl), 3,40-3,75 (2 H, CH₂-O-vinyl + 2 H, CH₂-O-glyceryl + 1 H, CH-OH + 1 H, CH₂-OH + O-CH₂-CHOH + 2x 1 H, OH), 3,85 (1 H, CH₂-OH), 3,99 (1 H, CH₂=CH-O trans), 4,19 (1 H, CH₂=CH-O cis), 6,47 (1 H, CH₂=CH-O gemi), keine verbliebenen Epoxid-peaks wurden beobachtet.

### Beispiel 4a: Synthese von 4-Glycidylbutylvinylether (GBVE)

116,16 g (1 mol) HBVE und 10,51 (0,05 mmol) Tetrabutylammoniumbromid wurden in einem 1 I Rundkolben mit einem Tropftrichter mit Druckausgleich vorgelegt. Eine Mischung aus 300 ml Toluol und 300 ml 50 %-iger wässriger Natriumhydroxid-Lösung wurde zugefügt. Die Reaktionsmischung wurde mit einem Eisbad gekühlt und schnell gerührt. 148,16 g (2 mol) Epichlorhydrin wurden langsam zugefügt und die resultierende Emulsion über einen Zeitraum von 16 h bei Raumtemperatur gerührt. Die organische Phase wurde mehrere Male mit gesättigter Natriumchlorid-Lösung und Wasser gewaschen. Lösemittel wurde unter vermindertem Druck entfernt und das Produkt mittels Vakuumdestillation aufgereinigt, um eine farblose Flüssigkeit zu erhalten. Ausbeute: 66 %. ¹H-NMR (CDCl₃, 400 MHz): δ (ppm) = 1,6-1,8 (4 H, mid-CH₂ Butyl), 2,60 (1 H, CH₂ Epoxid), 2,79 (1 H, CH₂ Epoxid), 3,14 (1 H, CH Epoxid), 3,38 (1 H, CH₂ Glycidylether), 3,53 (2 H, CH₂-O-glycidyl), 3,65-3,75 (2 H, CH₂-O-vinyl + 1 H, CH₂ Glycidylether), 3,97 (1 H, CH₂=CH-O trans), 4,17 (1 H, CH₂=CH-O cis), 6,47 (1 H, CH₂=CH-O gemi).

### Beispiel 4b: Synthese von 4-Glycidylcarbonatbutylvinylether (GCBVE)

4-Glycidylcarbonatbutylvinylether (GCBVE) wurde via CO₂-Insertion in 17,22 g (0,1 mol) 4-Glycidylbutylvinylether in einem Verfahren wie in der Literatur beschrieben (Poly. Chem., 2013, 4, S. 4545-4561) synthetisiert. Ausbeute: 87 %. ¹H-NMR (CDCl₃, 400 MHz): δ (ppm) = 1,6-1,8 (4 H, mid-CH₂ Butyl), 3,55 (2 H, CH₂-O-glycidylcarbonat), 3,62 (1 H, CH₂-carbonat), 3,70 (2 H, CH₂-O-vinyl + 1 H, CH₂-carbonat), 3,99 (1 H, CH₂=CH-O trans), 4,19 (1 H, CH₂=CH-O cis), 4,39 (1 H, CH₂ Carbonat), 4,50 (1 H, CH₂ Carbonat), 4,82 (1 H, CH Carbonat), 6,46 (1 H, CH₂=CH-O gemi), 2,5-3,5 (CH₂/CH Epoxid). Integration zeigt <2% verbliebenes Epoxy.

### Beispiel 5: Polyol-Synthese durch Ring-Öffnung von GCBVE mit Hexamethylendiamin

10,81 g (50 mmol) GCBVE und 2,95 g (25 mmol) Hexamethylendiamin wurden in einem Rundkolben vorgelegt und für 9 h auf 80 °C erhitzt. Umsatz wurde via Verschwinden der Carbonat-C=O-Valenzschwingungsbande im IR-Spektrum beobachtet. Ausbeute: quantitativ.

### Beispiel 6: Synthese von Vinylether-funktionalisierten Polyurethanen

Die Polyurethane wurden in Ansätzen zu jeweils 15-40 g synthetisiert. Die Stöchiometrie wurde so berechnet, dass ein NCO-terminiertes Präpolymer mit einem zahlenmittleren Molekulargewicht von Mₙ = 5000 g/mol erhalten wurde. Die Polyole wurden in einem kleinen Rundkolben im Vakuum bei 75 °C getrocknet. Anschließend wurden die Isocyanatverbindungen bei 40 °C hinzugefügt. Eine Probe der Mischung wurde für IR-spektroskopische Untersuchungen entnommen. Die der N=C=O Valenzschwingung entsprechende Bande bei ungefähr 2550 cm⁻¹ wurde integriert und mit der ursprünglichen Konzentration der Isocyanatgruppen korreliert. Anschließend wurde der Katalysator (50 mg/100 g Produkt, als 50 %-ige Lösung in trockenem Aceton) zugefügt und die Mischung wurde vorsichtig auf 80 °C erhitzt. Nach einer Stunde Reaktionszeit wurde ein Aliquot entnommen, um die gewünschte Isocyanatkonzentration via IR-Spektroskopie zu bestätigen. 90 % der stöchiometrischen Menge des end-capping Agens wurde hinzugefügt, um einen Überschuss an Hydroxylgruppen im Produkt zu vermeiden, und nach 30 min wurde eine weitere Probe entnommen, um einen fast vollständigen Umsatz des Isocyanats mittels IR-Spektroskopie zu bestätigen. Das Produkt wurde schließlich mit trockenem Aceton zu 50 Gew.-% Polyurethangehalt verdünnt. Ausbeute: 95 %.

### Beispiel 7: Synthese eines Vinylether-functionalisierten Polyurethans

10.00 g des in Beispiel 1 synthetisierten Vinyletherpolyols wurden unter reduziertem Druck bei 75°C entgast. Bei 40°C wurden dann 5,82 g Isophorondiisocyanat (Merck, 99%) und 0,0162 g Fomrez Katalysator UL-28 (Momentive) zugegeben und die Mischung langsam auf 80°C erwärmt. Es wurde ein Seitenketten-Vinylether-funktionalisiertes Polyurethanpräpolymer (sc-VEPU) erhalten. Nach 1 h wurden 0.62 g 4-Hydroxybutylvinylether zugegeben und die Reaktionsmischung für weitere 30 Minuten gerührt, um die terminalen Isocyanatgruppen ebenfalls mit HBVE umzusetzen und dadurch zusätzlich terminale Vinylethergruppen zu erzeugen. Die Synthese ist in Figur 1 schematisch dargestellt. Bei einem Mn = 5000 g/mol wurde eine durchschnittliche Vinyletherfunktionalität von etwa 16,5 erhalten.

Zu Vergleichszwecken wurden ein Vinylether-terminiertes Polyurethan (t-VEPU) und ein inaktives Alkyl-terminiertes Polyurethan (i-PU) aus IPDI und Polypropylenglykol (PPG) (Dow Chemical, Voranol 2000 L, 2000 g/mol) unter Verwendung von 1-Heptanol bzw. HVBE als EndgruppenVerkappungsmittel synthetisiert. Für t-VEPU wurde damit eine Vinyletherfunktionalität von 2 erhalten

Die Härtung erfolgte wie folgt: 1,98 g des mit Vinylether-Seitenketten funktionalisierten Polyurethans (sc-VEPU) wurden mit 0,02 g Omnicat 440 (IGM) und 2 g Aceton (Lösungsmittel) versetzt, wobei letzteres im Folgenden unter reduziertem Druck entfernt wurde. Die Formulierung wurde als dünner Film auf eine Oberfläche appliziert und unter UV-Bestrahlung (Omnicure S2000SC, 10 s) ausgehärtet, um eine klebefreien Film zu ergeben. Der auf eine Glasoberfläche applizierte und gehärtete sc-VEPU Film ist in Fibur 2b gezeigt. Das Bild zeigt, dass sich so farblose und hochtransparente Filme erzeugen lassen.

### Beispiel 8: Synthese eines hydrierten Vinyletherpolyols (hsc-VEPU)

Eine Lösung des VEOH aus Beispiel 1 (0,02 mol/L) in Methanol wurde unter Verwendung einer H-Cube Vorrichtung HC-2.SS zur kontinuierlichen Hydrierung (ThalesNano) hydriert. Die erfoderliche Menge an Wasserstoff wurde durch Elektrolyse von Wasser erzeugt und anschließend getrocknet. Die Lösung des Reaktanden wurde dann mit Wasserstoff bei einem Druck von 20 bar bei 25°C in einer Mischkammer beladen und mit einer konstanten Flußrate von 1.2 ml/min durch die Reaktionskammer, die eine 10% Pd/C (CatCart 30) Katalysatorkartusche enthielt geleitet. Methanol wurde unter reduziertem Druck entfernt. Ausbeute: 98%. ¹H-NMR (CDCl₃, 400 MHz): δ (ppm) = 1.2 (6H, CH₃), 1.6-1.8 (12H, mid-CH₂ butyl), 3.4-3.55 (24H, CH₂-O-CH₂), 3.93 (2H, CH-O), 4.19 & 6.46 (restlicher Vinylether, Peak Integration zeigte 1-2% Rückstand). Die Synthese ist in Figur 1 schematisch dargestellt.

Danach wurde 4-Hydroxybutylvinylether zugegeben und die Reaktionsmischung für weitere 30 Minuten gerührt, um die terminalen Isocyanatgruppen mit HBVE umzusetzen und dadurch terminale Vinylethergruppen zu erzeugen. Für hsc-VEPU wurde damit eine Vinyletherfunktionalität von 2 erhalten.

### Beispiel 9: UV-NIR Rheometrie

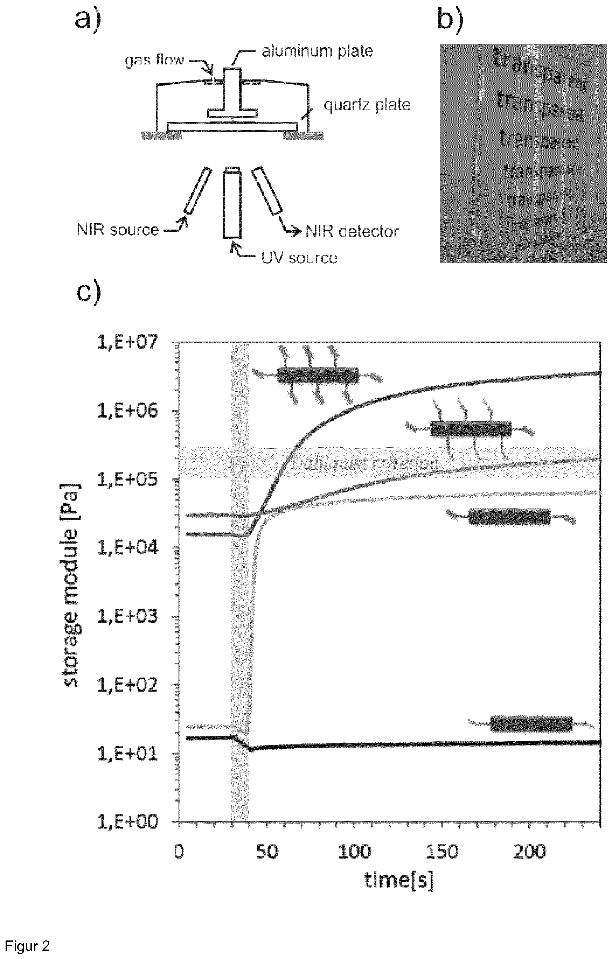

Die glechzeitige Messung der viskoelastischen Eigenschaften und Aufnahme von Nah-Infrarot (NIR) Spektren nach UV-Initiierung wurde mit einem Rheometer und einem Versuchsaufbau durchgeführt wie von Scherzer beschrieben (Scherzer, T.; Schröder, M. W. Proc. RadTech Europe 2009 Conference 2009**).** Ein Anton Paar MCR 302 Rheometer wurde in Verbindung mit einem Bruker MPA FT-NIR Spektrometer und einer Omnicure S 2000 SC Lichtquelle verwendet, wobei beide durch die Rheometer Software ausgelöst wurden. Der Versuchsaufbau ist in Figur 2a schematisch dargestellt. Die Probe wurde im Zentrum der Quarzbodenplatte platziert und eine Aluminiumplatte mit 20 mm Durchmesser wurde als bewegliche Spindel mit einem anfänglichen Spalt von 0,3 mm verwendet. Für die automatische Spaltkontrolle bei Schrumpfung der Probe wurde eine Normalkraft von Null verwendet, um Delamination zu vermeiden. Die mechanischen Daten wurden über Oszillation der Spindel erhalten. Es wurde ein ansteigendes Messprofil verwendet, um lineares viskoelastisches Verhalten sicherzustellen und innerhalb der Instrumentenbeschränkungen zu bleiben, da die Proben Moduli bei der Härtung um mehrere Größenordnungen ansteigen. Vor der UV-Bestrahlung wurde eine sinusförmige Zugspannung von 10% für 30 s mit einer Frequenz von 10 Hz angelegt. Bei der UV-Bestrahlung, die durch die transparente Bodenplatte durchgeführt wurde, wurde die Zugspannung linear innerhalb von 10 Sekunden von 10% auf 0.1% reduziert und mechanische Daten mit einer Rate von 1 s⁻¹ aufgenommen (auf das inaktive Heptanol-verkappte Polyurethan wurde kein ansteigendes Messprofil verwendet). Die UV-Lichtquelle wurde so eingestellt, dass sie die Probe 10 Sekunden lang bestrahlte. Die Intensität von 2 mW cm⁻² UVC (189 mW cm⁻² UVA-C) an der Oberfläche der Quarzplatte wurde regelmäßig mittels eines Spektralradiometers (OpSyTec Dr. Göbel) kontrolliert.

Nach der UV-Bestrahlung wurde der Nachhärtungsverlauf für 200 s bei einer Zugspannung von 0.1% aufgenommen. Einige Proben wurden dann für weitere 10 s bestrahlt und erneut die Nachhärtung aufgenommen. Es wurde kein Versuch unternommen, gelöste Gase aus den Proben zu entfernen und die Messungen wurden unter instrumenteller Luftatmospähre (H₂O: 1.1 mg/m³) aufgenommen. Zu Vergleichszwecken wurde ein Experiment unter Stickstoffatmosphäre durchgeführt (N₂: <99.9996%, O₂: <0.5 ppm, H₂O: <1 ppm). Die NIR Spektren wurden mit einer Auflösung von 16 cm⁻¹ bei einer konstanten Aufnahmerate von ungefähr 2 Spektren s⁻¹ aufgenommen. Die relative Vinyletherkonzentration wurde aus der integrierten Peakfläche der C=C-Dehnungsvibration bei ungefähr 6200 cm⁻¹ berechnet. Der Mittelwert für diesen Peak in den Spektren vor der Bestrahlung wurde auf 100% gesetzt.

Figur 2c zeigt die rheometrischen Plots für die synthetisierten Polyurethane i-PU, t-VEPU, sc-VEPU und hsc-VEPU (siehe Beispiele 6-8). Der Bestrahlungszeitraum von 10 Sekunden ist durch den grau hinterlegten Bereich in dem Diagramm gekennzeichnet. Wie erwartet zeigte i-PU keinen Anstieg des Speichermoduls. Die leichte Abnahme des Speichermoduls bei der Bestrahlung in allen Proben ist vermutlich ein thermischer Effekt aufgrund von Lichtabsorption oder dem Abbau des Photoinitiators, wobei die abgespaltenen Initiatorfragmente als Weichmacher wirken können. Das t-VEPU zeigt eine Verzögerung von ca. 10 s zwischen Bestrahlung und dem anfänglichen, schnellen Anstieg des Speichermoduls. Bei der Härtung des terminal funktionalisierten PUs unter trockenem Stickstoff um jeden Einfluss von Sauerstoff oder Luftfeuchtigkeit auszuschließen wurde keine Verbesserung beobachtet. Die Verzögerung ist vermutlich auf eine relativ langsame Anfangsreaktion zurückzuführen und ein ähnliches Verhalten wurde für die Seitenketten-funktionalisierten Proben beobachtet. Das t-VEPU besitzt aufgrund seiner chemischen Struktur ein relativ niedriges Start-Speichermodul, da es eine geringe Menge an Urethanbindungen und ungefähr 82 Gew.-% PPG Segmente aufweist. Bei UV-Bestrahlung kann das Speichermodul daher um mehrere Größenordnungen ansteigen, aber übersteigt nicht das Dahlquist-Kriterium, das den Grenzwert angibt über dem klebefreie Filme erhalten werden (Dahlquist, C. A., Tack. In Adhesion Fundamentals and Practice, 1969; pp 143-151). Die Klebrigkeit des gehärteten t-VEPUs zeigt, dass die flexiblen Polymerketten über die terminalen, reaktiven Gruppen nicht stark genug quervernetzt werden können. Die erste Oberschwingung der Dehnschwingung der Vinylether C=C-Doppelbindung kann als relativ scharfe Absorptionsbande bei 6200 cm⁻¹ im NIR-Spektrum gefunden werden. (Workman, J.; Weyer, L., Alkenes and Alkynes. In Practical Guide and Spectral Atlas for Interpretive Near-Infrared Spectroscopy, Second Edition, CRC Press: 2012; pp 33-38.; Scherzer, T.; Buchmeiser, M. R. Macromolecular Chemistry and Physics 2007, 208, (9), 946-954). Die Analyse der integrierten Peakfläche zeigt den Verbrauch des Vinylethers. Das Signal-Hintergrundverhältnis war aufgrund der niedrigen Konzentration der Endgruppen allerdings zu niedrig, um verläßlich die Konversion zu berechnen.

Das hsc-VEPU, das ebenfalls nur terminale Vinylethergruppen trägt, wurde hergestellt, um den Einfluss des Polyurethanrückgrats zu untersuchen. Die kurze Polyolstruktur des hydrierten VEOH verschiebt die Zusammensetzung in Richtung eines höheren Gehalts an harten Urethansegmenten, was zu stärkeren intermolekularen Wechselwirkungen und höherer Anfangsviskosität führt.

Dementsprechend ist das Speichermodul zu Beginn signifikant höher und entwickelt sich nach UV-Initiierung mit einer niedrigeren Rate. Das ist auf eine verringerte Mobilität der funktionellen Gruppen und langsamere Diffusionskinetiken zurückzuführen. Insbesondere Makromonomere werden durch erhöhte Viskositäten stark beeinflusst. Auf der anderen Seite zeigt die langsamere Reaktion deutlich die Nachhärtung Obwohl das ausgehärtete hsc-VEPU ein größeres Speichermodul zeigt als t-VEPU, ist es immer noch leicht klebrig.

Im Gegensatz härtet das hochgradig Vinylether-funktionalisierte sc-VEPU mit einer vergleichbaren Rückgratstruktur zu einem klebefreien Film aus und zeigt als direkte Konsequenz der erzielbaren hohen Quervernetzungsraten ein überragendes Speichermodul, was insbesondere für die Eignung als Baumaterial für Bedeutung ist.

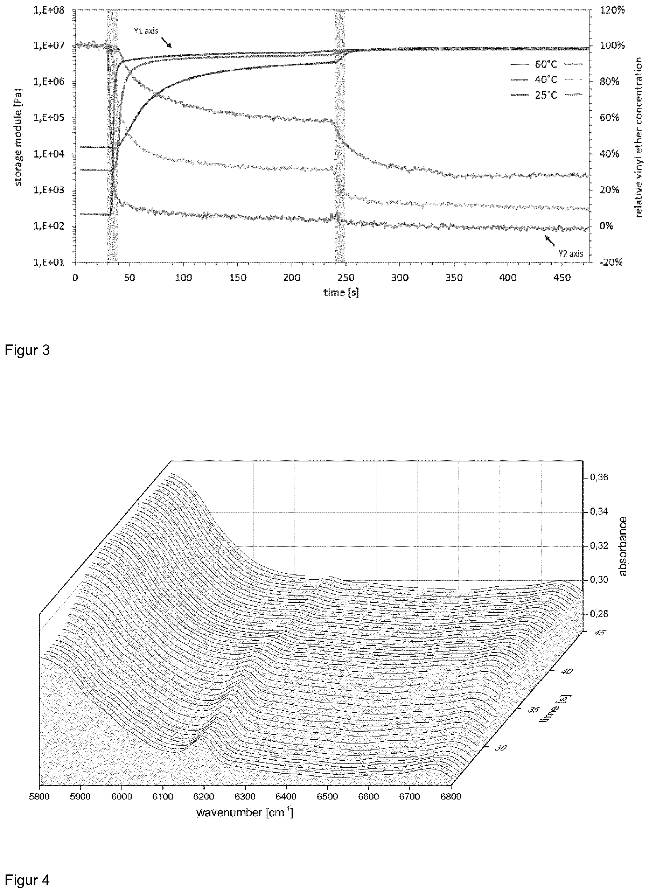

Figur 3 zeigt die Aushärtung von Seitenketten-funktionalisiertem Polyurethan (sc-VEPU) über die Entwicklung der Speichermodule und relativen Vinylethergehalte mittels in-situ NIR Messung bei unterschiedlichen Temperaturen (25°C, 40°C and 60°C), wobei diese bei höheren Temperaturen beschleunigt wird. Bei 25°C und 40°C werden nach einer ersten Initiierung Konversionsraten von ungefähr 45% bzw. 75% erreicht. Eine zweite Initiierung kann die Konversion auf ungefähr 70% bzw. 90% erhöhen. Es wird angenommen, dass die aktiven Kettenenden in quervernetzten Regionen eingeschlossen werden und dadurch für restliche Vinylethergruppen unzugänglich werden. Die zweite Initiierung erzeugt neue polymerisierbare Gruppen die dann zu diesem Zeitpunkt weniger quervernetzt und mobiler sind. Eine bemerkenswerte und mechanistisch wichtige Beobachtung ist, dass bei 60°C die sc-VEOH Polymerisation sehr schnell bis zur nahezu vollständigen Konversion abläuft. Ein starker Einfluss durch die Terminierung kann somit ausgeschlossen werden, da die entsprechenden Terminierungsreaktionen höhere Aktivierungsenergien besitzen als die fortschreitende Reaktion und bei erhöhten Temperaturen somit stärker beschleunigt würden.

Leicht negative Werte für die Rest-Vinylether-Konzentration in Figur 3 stammen aus dem vollständigen Verschwinden des Peaks in einem konvexen Bereich der Spektren (siehe Figur 4). Figur 4 zeigt die NIR-Spektren des sc-VEPU (Beispiel 7) bei UV-initiierter Härtung bei 60°C. Die NIR-Spektren wurden mit dem beschriebenen UV-NIR-Rheometer Aufbau aufgenommen, wobei die Probe 30 - 40 s bestrahlt wurde. Die ansteigende Gesamtintensität zu diesem Zeitpunkt korreliert mit der Schrumpfung der Probe um 3.7%. Der Peak bei 6200 cm⁻¹ wird der ersten Oberschwingung der C-H Dehnschwingung zugeordnet und nimmt bei der Polymerisation nach UV-Initiierung ab.

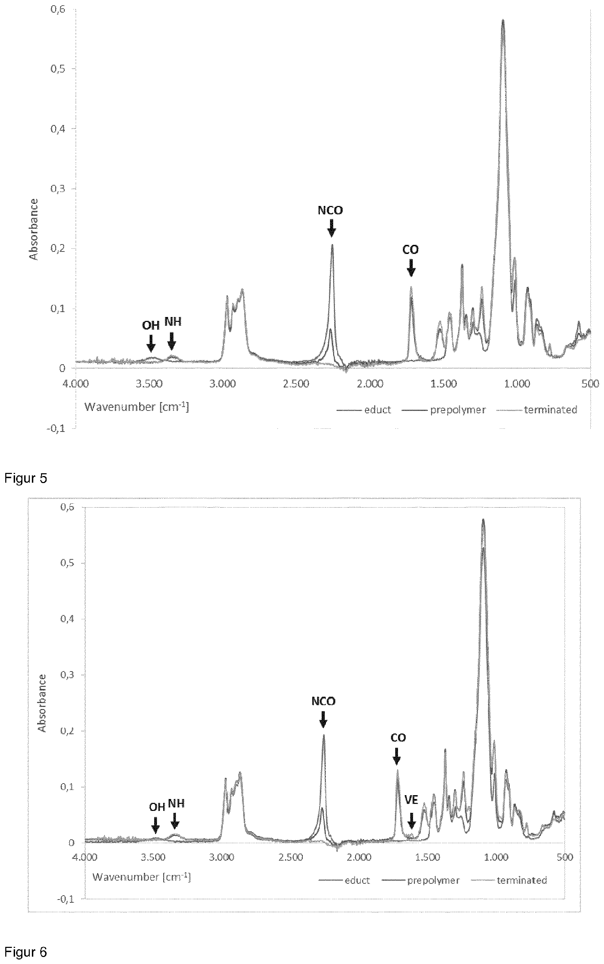

Die Ergebnisse der Messungen sind in den Figuren 5-8 gezeigt. Dabei zeigt Figur 5 das IR-Spektrum der i-PU-Synthese (inaktiv-Alkyl-terminiertes Pulyurethan). Dieses Polyurethan wurde ausgehend von Isophorondiisocyanat (IPDI) und Polypropylenglykol (PPG) nach der Vorschrift in Beispiel 6 synthetisiert und mit 1-Heptanol terminiert (Figur 1). Spektren wurden nach Zugabe des Isocyanats (Edukt), nach einer Stunde Reaktionszeit (Präpolymer) und nach 30 min nach Zugabe des Terminierungsagens (terminiert) aufgenommen. Einige strukturrelevante Peaks sind zugeordnet. Die integrierten Peakflächen, die der N=C=O Valenzschwingung bei 2550 cm⁻¹ entsprechen, sowie die dazugehörigen molaren Mengen, die aus der Stöchiometrie berechnet wurde, sind wie folgt:

| | n(NCO) | | A(NCO) | |
|---|---|---|---|---|
| | [mmol] | % | [Counts] | % |
| Edukt | 45.0 | 100 | 9567 | 100 |
| Präpolymer | 14.3 | 32 | 3005 | 31 |
| terminiert | 1.4 | 3 | 276 | 3 |

Figur 6 zeigt das IR-Spektrum der t-VEPU-Synthese (Vinylether-terminiertes Polyurethan). Das Polyurethan wurde ausgehend von IPDI und PPG nach der Vorschrift in Beispiel 6 synthetisiert und mit 4-Hydroxybutylvinylether terminiert (Figur 1). Spektren wurden nach Zugabe des Isocyanats (Edukt), nach einer Stunde Reaktionszeit (Präpolymer) und nach 30 min nach Zugabe des Terminierungsagens (terminiert) aufgenommen. Einige strukturrelevante Peaks sind zugeordnet. Die integrierten Peakflächen, die der N=C=O Valenzschwingung bei 2550 cm⁻¹ entsprechen, sowie die dazugehörigen molaren Mengen, die aus der Stöchiometrie berechnet wurde, sind wie folgt:

| | n(NCO) | | A(NCO) | |
|---|---|---|---|---|
| | [mmol] | % | [Counts] | % |
| Edukt | 45.0 | 100 | 9839 | 100 |
| Präpolymer | 14.3 | 32 | 3022 | 31 |
| terminiert | 1.4 | 3 | 358 | 4 |

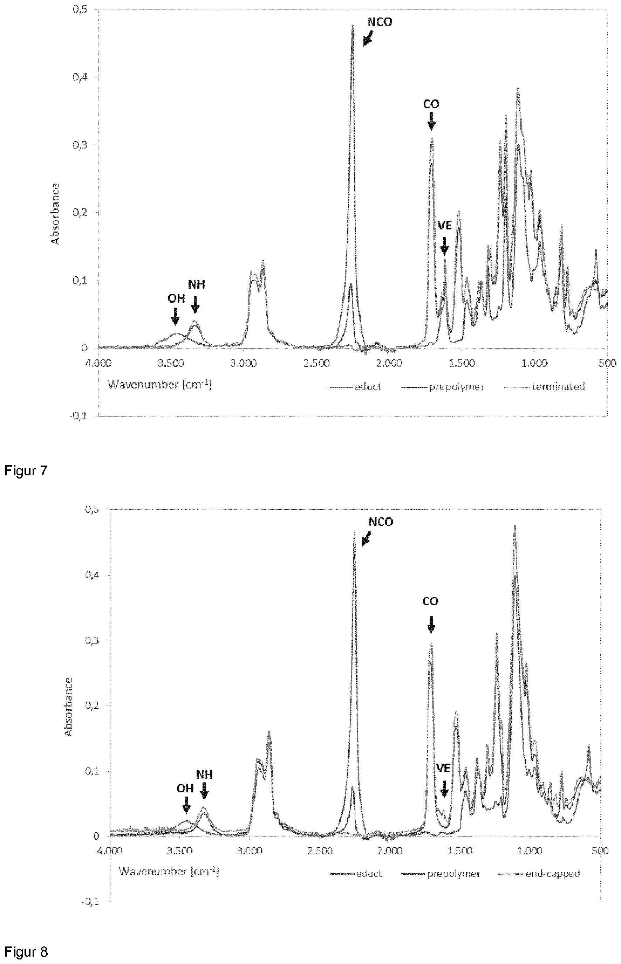

Figur 7 zeigt das IR-Spektrum der sc-VEPU-Synthese (Seitenketten Vinylether-funktionalisiertes Polyurethan). Das Polyurethan wurde ausgehend von IPDI und VEOH nach der Vorschrift in Beispiel 7 synthetisiert und mit 4-Hydroxybutylvinylether terminiert (Figur 1). Spektren wurden nach Zugabe des Isocyanats (Edukt), nach einer Stunde Reaktionszeit (Präpolymer) und nach 30 min nach Zugabe des Terminierungsagens (terminiert) aufgenommen. Einige strukturrelevante Peaks sind zugeordnet. Die C=C Dehnschwingungsbande des Vinylethers bei 1615 cm⁻¹ zeigt eindeutig eine vergleichsweise hohe Vinyletherkonzentration und bestätigt, dass unter Synthesebedingungen kein Verbrauch des Vinylethers stattfindet.Die integrierten Peakflächen, die der N=C=O Valenzschwingung bei 2550 cm⁻¹ entsprechen, sowie die dazugehörigen molaren Mengen, die aus der Stöchiometrie berechnet wurde, sind wie folgt:

| | n(NCO) | | A(NCO) | |
|---|---|---|---|---|
| | [mmol] | % | [Counts] | % |
| Edukt | 52.4 | 100 | 26020 | 100 |
| Präpolymer | 6.3 | 12 | 4600 | 18 |
| terminiert | 0.6 | 1 | 294 | 1 |

Es zeigt sich, dass die Reaktion unter den gegebenen Bedingungen gut kontrolliert werden konnte.

Figur 8 zeigt das IR-Spektrum der hsc-VEPU-Synthese (hydriertes Seitenketten Vinylether-funktionalisiertes Polyurethan). Das Polyurethan wurde ausgehend von IPDI und VEOH nach der Vorschrift in Beispiel 7 synthetisiert und mit 4-Hydroxybutylvinylether terminiert. Anschließend wurde es hydriert (Figur 1). Spektren wurden nach Zugabe des Isocyanats (Edukt), nach einer Stunde Reaktionszeit (Präpolymer) und nach 30 min nach Zugabe des Terminierungsagens (terminiert) aufgenommen. Einige strukturrelevante Peaks sind zugeordnet. Die C=C Dehnschwingungsbande des Vinylethers bei 1615 cm⁻¹ zeigt, dass das hsc-VEPU nur geringe Spuren an verbliebenen Vinylethergruppen aufweist. Die integrierten Peakflächen, die der N=C=O Valenzschwingung bei 2550 cm⁻¹ entsprechen, sowie die dazugehörigen molaren Mengen, die aus der Stöchiometrie berechnet wurde, sind wie folgt:

| | n(NCO) | | A(NCO) | |
|---|---|---|---|---|
| | [mmol] | % | [Counts] | % |
| Edukt | 52.4 | 100 | 25601 | 100 |
| Präpolymer | 6.3 | 12 | 3277 | 13 |
| terminiert | 0.6 | 1 | 201 | 1 |

## Patentansprüche

1. Verfahren zur Herstellung eines Alkenyletherpolyols enthaltend mindestens eine Alkenylethergruppe, insbesondere eine 1-Alkenylethergruppe, und mindestens zwei Hydroxylgruppen (-OH),
durch
A) Umsetzen eines Alkenylethers, enthaltend mindestens eine Alkenylethergruppe und mindestens eine funktionelle Gruppe ausgewählt aus -OH, -COOH, -SH, -NH₂ und deren Derivaten,
mit (i) einem Epoxid oder (ii) einem cyclischen Carbonat oder Derivat davon; oder
B) Umsetzen eines Alkenylethers, enthaltend mindestens eine Alkenylethergruppe und mindestens eine funktionelle Gruppe ausgewählt aus (i) Epoxidgruppen und (ii) cyclischen Carbonatgruppen oder Derivaten davon,
mit einem Alkohol, Thiol, einer Carbonsäure oder einem Amin oder Derivaten der vorgenannten.

2. Verfahren nach Anspruch 1, wobei das Alkenyletherpolyol hergestellt wird durch Umsetzen eines Alkenylethers, enthaltend mindestens eine Alkenylethergruppe und mindestens eine funktionelle Gruppe ausgewählt aus -OH, -COOH, -SH, -NH₂ und deren Derivaten, mit (i) einem Epoxid oder (ii) einem cyclischen Carbonat oder Derivat davon, **dadurch gekennzeichnet, dass** das Alkenyletherpolyol ein Alkenyletherpolyol der Formel (I) ist wobei 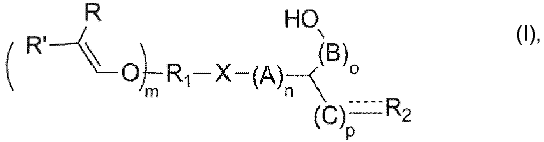
R₁ ein mindestens 2-wertiges lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom,
R₂ ein, optional 2- oder mehrwertiges, lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom, jeweils optional mit mindestens einer -OH Gruppe, oder ein (Poly)alkylenglykol, insbesondere ein (Poly)alkylenglykol der Formel -O-[CHRₐCH₂O]_{b}-R_{b}, wobei Rₐ H oder ein C₁₋₄-Alkylrest, R_{b} -H und b 1 bis 100 ist,
X O, S, C(=O)O, OC(=O)O, C(=O)OC(=O)O, NRₓ, NRₓC(=O)O, NRₓC(=O)NRₓ oder OC(=O)NRₓ ist,
jedes R und R' unabhängig ausgewählt ist aus H, C₁₋₂₀ Alkyl und C₂₋₂₀ Alkenyl, wobei insbesondere eines von R und R' H und das andere C₁₋₄ Alkyl oder beide H sind,
jedes A, B und C unabhängig ausgewählt ist aus CR"R''',
R" und R''' unabhängig ausgewählt sind aus H, einer funktionellen Gruppe, und einem organischen Rest, insbesondere H und C₁₋₂₀ Alkyl, oder R" und R''' gemeinsam oder mit dem Kohlenstoffatom an welches sie gebunden sind ein organischer Rest sind, oder zwei von R" und R''' die an benachbarte Kohlenstoffatome gebunden sind, zusammen eine Bindung bilden um eine Doppelbindung zwischen den benachbarten Kohlenstoffatomen auszubilden,
------ eine Einfach- oder Doppelbindung ist, wobei wenn es eine Doppelbindung ist,
das Kohlenstoffatom, das an R₂ gebunden ist nur einen Substituenten R" oder R'''
trägt,
m eine ganze Zahl von 1 bis 10, vorzugsweise 1, ist,
n, p und o jeweils 0 oder eine ganze Zahl von 1 bis 10 sind, wobei n+p+o=1 oder mehr ist, insbesondere 1 oder 2, und
Rₓ H, oder 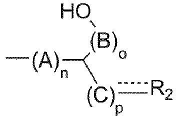 wobei wenn X nicht NRₓ mit Rₓ = 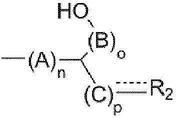 ist, R₂ mindestens einen Substituenten aufweist, der ausgewählt ist aus -OH und

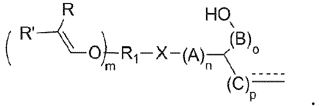

3. Verfahren nach Anspruch 2, **dadurch gekennzeichnet, dass** der Alkenylether, der mindestens eine Alkenylethergruppe und mindestens eine funktionelle Gruppe ausgewählt aus -OH, - COOH, -SH, -NH₂ und deren Derivaten enthält, ein Alkenylether der Formel (II) ist 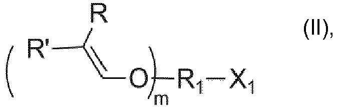 wobei
R₁ ein mindestens 2-wertiges lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom ist,
X₁ eine funktionelle Gruppe ausgewählt aus -OH, -COOH, -SH, -NHR_{y} und deren Derivaten,
R_{y} H oder ein organischer Rest,
jedes R und R' unabhängig ausgewählt ist aus H, C₁₋₂₀ Alkyl und C₂₋₂₀ Alkenyl, wobei insbesondere eines von R und R' H und das andere C₁₋₄ Alkyl oder beide H sind, und
m eine ganze Zahl von 1 bis 10, vorzugsweise 1, ist.

4. Verfahren nach Anspruch 3, **dadurch gekennzeichnet, dass** in dem Alkenylether der Formel (II) m 1 ist,
X₁ -OH oder -NH₂, vorzugsweise -OH, ist,
R₁ ein zweiwertiger, linearer oder verzweigter C₁₋₁₀ Alkylrest, insbesondere Ethyl, Propyl, Butyl, Pentyl oder Hexyl, ist, und
eines von R und R' H und das andere H oder -CH₃ ist.

5. Verfahren nach einem der Ansprüche 1 bis 4, **dadurch gekennzeichnet, dass** das Epoxid ein Epoxid der Formel (III) ist 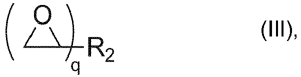 wobei R₂ ein, optional 2- oder mehrwertiges, lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom ist, optional mit mindestens einer -OH Gruppe, oder ein (Poly)alkylenglykol, insbesondere ein (Poly)alkylenglykol der Formel -O-[CHRₐCH₂O]_{b}-R_{b}, wobei Rₐ H oder ein C₁₋₄-Alkylrest, R_{b} -H und b 1 bis 100 ist, und
q eine ganze Zahl von 1 bis 10, vorzugsweise 1 oder 2, ist.

6. Verfahren nach Anspruch 5, **dadurch gekennzeichnet, dass** in dem Epoxid der Formel (III) q 1 oder 2 ist, und
wenn q 2 ist, R₂ -CH₂-O-C₁₋₁₀-Alkylenyl-O-CH₂- ist, und
wenn q 1 ist, R₂ -CH₂-O-C₁₋₁₀-Alkyl ist.

7. Verfahren nach einem der Ansprüche 1 bis 4, **dadurch gekennzeichnet, dass** das cyclische Carbonat ein Ethylencarbonat der Formel (IV) ist 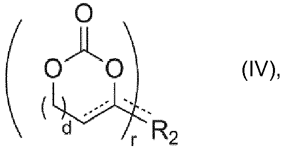
wobei R₂ ein, optional 2- oder mehrwertiges, lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom ist, optional mit mindestens einer -OH Gruppe, oder ein (Poly)alkylenglykol, insbesondere ein (Poly)alkylenglykol der Formel -O-[CHRₐCH₂O]_{b}-R_{b}, wobei Rₐ H oder ein C₁₋₄-Alkylrest, R_{b} -H und b 1 bis 100 ist, noch bevorzugter ein C₁₋₁₀ Hydroxyalkyl,
------ eine Einfach- oder Doppelbindung, vorzugsweise eine Einfachbindung, ist,
d 0 oder 1, vorzugsweise 0, ist, und
r eine ganze Zahl von 1 bis 10, vorzugsweise 1 oder 2, ist.

8. Verfahren nach einem der Ansprüche 3 bis 7, **dadurch gekennzeichnet, dass**
(i) X₁ -NH₂ oder ein Derivat davon ist, und
q oder r 1 ist;
(ii) X₁ -OH oder ein Derivat davon ist, und
q oder r 2 ist.

9. Verfahren nach Anspruch 1, wobei das Alkenyletherpolyol hergestellt wird Umsetzen eines Alkenylethers, enthaltend mindestens eine Alkenylethergruppe und mindestens eine funktionelle Gruppe ausgewählt aus (i) Epoxidgruppen und (ii) cyclischen Carbonatgruppen oder Derivaten davon, mit einem Alkohol, Thiol, einer Carbonsäure oder einem Amin oder Derivaten der vorgenannten, **dadurch gekennzeichnet, dass** das Alkenyletherpolyol ein Alkenyletherpolyol der Formel (V) ist 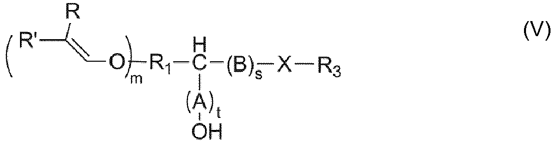 wobei
R₁ ein mindestens 2-wertiges lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom,
R₃ ein, optional 2- oder mehrwertiges, lineares oder verzweigtes, substituiertes oder unsubstituiertes Alkyl mit 1 bis 20 Kohlenstoffatomen, lineares oder verzweigtes, substituiertes oder unsubstituiertes Heteroalkyl mit 1 bis 20 Kohlenstoffatomen und mindestens einem Sauerstoff- oder Stickstoffatom, oder ein (Poly)Alkylenglykol der Formel -O-[CHRₐCH₂O]_{b}-R_{b} ist, wobei Rₐ H oder ein C₁₋₄-Alkylrest, R_{b} H oder 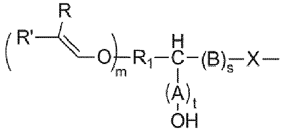 und b 1 bis 100 ist;
X O, S, OC(=O), OC(=O)O, OC(=O)OC(=O), NR_{z}, NR_{z}C(=O)O, NR_{z}C(=O)NR_{z} oder OC(=O)NR_{z} ist,
jedes R und R' unabhängig ausgewählt ist aus H, C₁₋₂₀ Alkyl und C₂₋₂₀ Alkenyl, wobei insbesondere eines von R und R' H und das andere C₁₋₄ Alkyl oder beide H sind,
jedes A und B unabhängig ausgewählt ist aus CR"R''',
R" und R''' unabhängig ausgewählt sind aus H, einer funktionellen Gruppe, und einem organischen Rest, insbesondere H und C₁₋₂₀ Alkyl, oder R" und R''' gemeinsam oder mit dem Kohlenstoffatom an welches sie gebunden sind ein organischer Rest sind, oder zwei von R" und R''' die an benachbarte Kohlenstoffatome gebunden sind, zusammen eine Bindung bilden um eine Doppelbindung zwischen den benachbarten Kohlenstoffatomen auszubilden,
m eine ganze Zahl von 1 bis 10, vorzugsweise 1, ist,
s und t jeweils 0 oder eine ganze Zahl von 1 bis 10 sind, wobei s+t=1 oder mehr ist, insbesondere 1 oder 2, und
R_{z} H oder 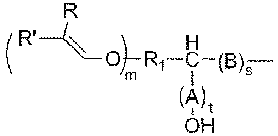 wobei wenn X nicht NR_{z} mit R_{z} = 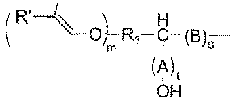 ist,
R₃ mindestens einen Substituenten aufweist, der ausgewählt ist aus -OH und

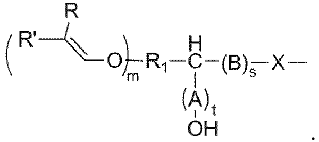

10. Verfahren nach Anspruch 9, **dadurch gekennzeichnet, dass** der Alkenylether, der mindestens eine Alkenylethergruppe und mindestens eine funktionelle Gruppe ausgewählt aus (i) Epoxidgruppen und (ii) cyclischen Carbonatgruppen oder Derivaten davon enthält, ein Alkenylether der Formel (VI) oder (VII) ist 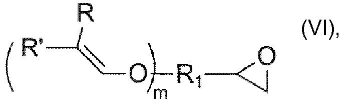 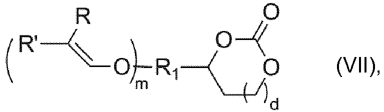 wobei R₁ ein mindestens 2-wertiges lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom ist,
jedes R und R' unabhängig ausgewählt ist aus H, C₁₋₂₀ Alkyl und C₂₋₂₀ Alkenyl, wobei insbesondere eines von R und R' H und das andere C₁₋₄ Alkyl oder beide H sind,
d 0 oder 1, vorzugsweise 0, ist, und
m eine ganze Zahl von 1 bis 10, vorzugsweise 1, ist.

11. Verfahren nach Anspruch 10, **dadurch gekennzeichnet, dass** in den Alkenylethern der Formel (VI) oder (VII)
R₁ -C₁-₁₀-Alkylenyl-O-CH₂- ist.

12. Verfahren nach Anspruch 10 oder 11, **dadurch gekennzeichnet, dass** der Alkenylether mit einem Alkohol umgesetzt wird, wobei es sich bei dem Alkohol um ein Diol oder Polyol oder ein entsprechendes Alkoholat, insbesondere ein Polyalkylenglykol der Formel HO-[CHRₐCH₂O]_{b}-H, wobei Rₐ H oder ein C₁₋₄-Alkylrest und b 1 bis 100, insbesondere 1 bis 10, ist, handelt.

13. Alkenyletherpolyol der der Formel (I) oder (V) 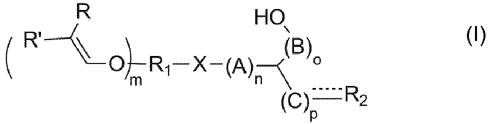 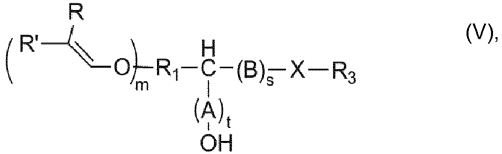 wobei
R₁ ein mindestens 2-wertiges lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom,
R₂ ein, optional 2- oder mehrwertiges, lineares oder verzweigtes, substituiertes oder unsubstituiertes Alkyl mit 1 bis 20 Kohlenstoffatomen oder lineares oder verzweigtes, substituiertes oder unsubstituiertes Heteroalkyl mit 1 bis 20 Kohlenstoffatomen und mindestens einem Sauerstoff- oder Stickstoffatom, optional mit mindestens einer -OH Gruppe, oder ein (Poly)alkylenglykol, insbesondere ein (Poly)alkylenglykol der Formel -O-[CHRₐCH₂O]_{b}-R_{b}, wobei Rₐ H oder ein C₁₋₄-Alkylrest, R_{b} -H und b 1 bis 100 ist,
R₃ ein, optional 2- oder mehrwertiges, lineares oder verzweigtes, substituiertes oder unsubstituiertes Alkyl mit 1 bis 20 Kohlenstoffatomen, lineares oder verzweigtes, substituiertes oder unsubstituiertes Heteroalkyl mit 1 bis 20 Kohlenstoffatomen und mindestens einem Sauerstoff- oder Stickstoffatom, oder ein (Poly)Alkylenglykol der Formel -O-[CHRₐCH₂O]_{b}-R_{b} ist, wobei Rₐ H oder ein C₁₋₄-Alkylrest, R_{b} -H oder 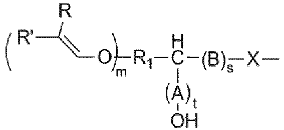 und b 1 bis 100 ist,
in Formel (I) X O, S, C(=O)O, OC(=O)O, C(=O)OC(=O)O, NRₓ, NRₓC(=O)O, NRₓC(=O)NRₓ oder OC(=O)NRₓ ist,
in Formel (V) X O, S, OC(=O), OC(=O)O, OC(=O)OC(=O), NR_{z}, NR_{z}C(=O)O, NR_{z}C(=O)NR_{z} oder OC(=O)NR_{z} ist,
jedes R und R' unabhängig ausgewählt ist aus H, C₁₋₂₀ Alkyl und C₂₋₂₀ Alkenyl, wobei insbesondere eines von R und R' H und das andere C₁₋₄ Alkyl oder beide H sind,
jedes A, B und C unabhängig ausgewählt ist aus CR"R''',
R" und R''' unabhängig ausgewählt sind aus H, einer funktionellen Gruppe, und einem organischen Rest, insbesondere H und C₁₋₂₀ Alkyl, oder R" und R''' gemeinsam oder mit dem Kohlenstoffatom an welches sie gebunden sind ein organischer Rest sind, oder zwei von R" und R''' die an benachbarte Kohlenstoffatome gebunden sind, zusammen eine Bindung bilden um eine Doppelbindung zwischen den benachbarten Kohlenstoffatomen auszubilden,
------ eine Einfach- oder Doppelbindung ist, wobei wenn es eine Doppelbindung ist,
das C, das an R₂ gebunden ist nur einen Substituenten R" oder R''' trägt,
m eine ganze Zahl von 1 bis 10, vorzugsweise 1, ist,
n, p und o jeweils 0 oder eine ganze Zahl von 1 bis 10 sind, wobei n+p+o=1 oder mehr ist, insbesondere 1 oder 2,
s und t jeweils 0 oder eine ganze Zahl von 1 bis 10 sind, wobei s+t=1 oder mehr ist, insbesondere 1 oder 2,
Rₓ H oder 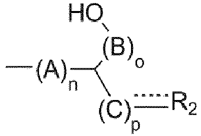 wobei wenn X nicht NRₓ mit Rₓ = 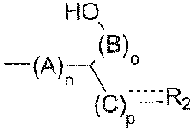 ist, R₂ mindestens einen Substituenten aufweist, der ausgewählt ist aus 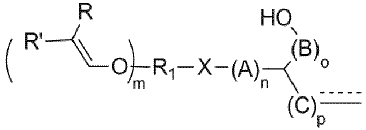 und
R_{z} H oder 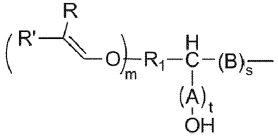 wobei wenn X nicht NR_{z} mit R_{z} = 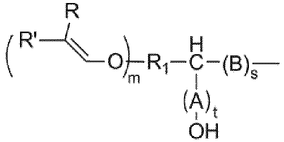 ist,
R₃ mindestens einen Substituenten aufweist, der ausgewählt ist aus -OH und

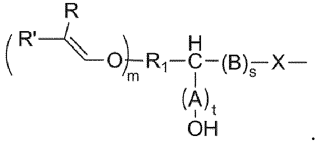

14. Alkenyletherpolyol nach Anspruch 13, **dadurch gekennzeichnet, dass** es erhältlich ist nach einem Verfahren gemäß einem oder mehreren der Ansprüche 1 bis 12.

15. Verwendung eines Alkenyletherpolyols nach Anspruch 13 oder 14 zur Synthese von strahlenvernetzbaren Oligomeren oder Polymeren mittels Polyadditions- oder Polykondensationsreaktionen, insbesondere zur Synthese von UV und EB--härtbaren Polyestern, Polyethern, Polyurethanen und Polyharnstoffen, besonders bevorzugt UV-härtbaren Polyurethanen.

16. UV-härtbares Polyurethanpolymer erhältlich durch Umsetzen mindestens eines Alkenyletherpolyols nach Anspruch 13 oder 14 mit einem Polyisocyanat.

## Claims

1. A method for preparing an alkenyl ether polyol containing at least one alkenyl ether group, in particular a 1-alkenyl ether group, and at least two hydroxyl groups (-OH),
by:
A) reacting an alkenyl ether, containing at least one alkenyl ether group and at least one functional group selected from -OH, -COOH, -SH, -NH₂ and derivatives thereof,
with (i) an epoxide or (ii) a cyclic carbonate or derivative thereof; or
B) reacting an alkenyl ether, containing at least one alkenyl ether group and at least one functional group selected from (i) epoxide groups and (ii) cyclic carbonate groups or derivatives thereof,
with an alcohol, thiol, a carboxylic acid, or an amine or derivatives thereof.

2. The method according to claim 1, in which the alkenyl ether polyol is prepared by reacting an alkenyl ether, containing at least one alkenyl ether group and at least one functional group selected from -OH, -COOH, -SH, -NH₂ and derivatives thereof, with (i) an epoxide or (ii) a cyclic carbonate or derivative thereof, **characterized in that** the alkenyl ether polyol is an alkenyl ether polyol of formula (I) 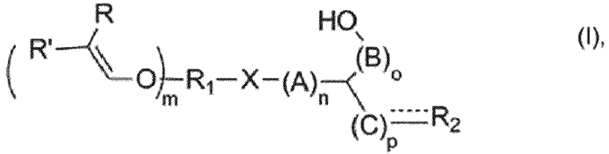 where
R₁ is an at least divalent linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom,
R₂ is an optionally divalent or polyvalent, linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom, in each case optionally having at least one -OH group or a (poly)alkylene glycol, in particular a (poly)alkylene glycol of the formula -O-[CHRₐCH₂O]_{b}-R_{b}, where Rₐ is H or a C₁₋₄ alkyl radical, R_{b} is -H and b is from 1 to 100,
X is O, S, C(=O)O, OC(=O)O, C(=O)OC(=O)O, NRₓ, NRₓC(=O)O, NRₓC(=O)NRₓ or OC(=O)NRₓ, each R and R' is selected independently from H, C₁₋₂₀ alkyl, and C₂₋₂₀ alkenyl, in particular one of R and R' being H and the other being C₁₋₄ alkyl or both R and R' being H,
each A, B, and C is independently selected from CR"R''',
R" and R'" are selected independently from H, a functional group, and an organic radical, in particular H and C₁₋₂₀ alkyl, or R" and R'" together or with the carbon atom to which they are bonded are an organic radical, or two of R" and R'" that are bonded to adjacent carbon atoms together form a bond in order to form a double bond between the adjacent carbon atoms,
------ is a single or double bond, and if it is a double bond,
the carbon atom that is bonded to R₂ bears only one substituent R" or R''',
m is an integer of from 1 to 10, preferably 1,
n, p, and o are each 0 or an integer of from 1 to 10, where n + p + o = 1 or more, in particular 1 or 2, and
Rₓ is H, or 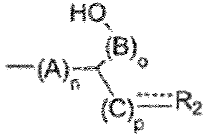 where, when X is not NRₓ, with Rₓ = 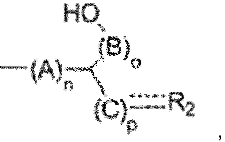 R₂ comprises at least one substituent, which is selected from -OH and

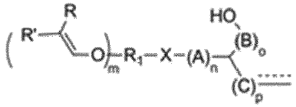

3. The method according to claim 2, **characterized in that** the alkenyl ether, which contains at least one alkylene ether group and at least one functional group selected from -OH, -COOH, -SH, - NH₂ and derivatives thereof, is an alkenyl ether of formula (II) 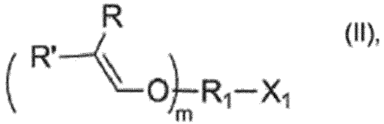 where
R₁ is an at least divalent linear or branched, substituted or unsubstituted alkyl having from 1 to 20 carbon atoms or linear or branched, substituted or
unsubstituted heteroalkyl having from 1 to 20 carbon atoms and at least one oxygen or nitrogen atom,
X₁ is a functional group selected from -OH, -COOH, -SH, -NHR_{y} and derivatives thereof, R_{y} is H or an organic radical,
each R and R' is selected independently from H, C₁₋₂₀ alkyl, and C₂₋₂₀ alkenyl, in particular one of R and R' being H and the other being C₁₋₄ alkyl or both R and R' being H, and
m is an integer of from 1 to 10, preferably 1.

4. The method according to claim 3, **characterized in that**, in the alkenyl ether of formula (II),
m is 1,
X₁ is -OH or -NH₂, preferably -OH,
R₁ is a divalent, linear or branched C₁₋₁₀ alkyl radical, in particular ethyl, propyl, butyl, pentyl, or hexyl, and
one of R and R' is H and the other is H or -CH₃.

5. The method according to one of claims 1 to 4, **characterized in that** the epoxide is an epoxide of formula (III) 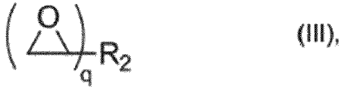 where R₂ is an optionally divalent or polyvalent, linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom, optionally comprising at least one -OH group, or a (poly)alkylene glycol, in particular a (poly)alkylene glycol of the formula -O-[CHRₐCH₂O]_{b}-R_{b}, where Rₐ is H or a C₁₋₄ alkyl radical, R_{b} is -H and b is from 1 to 100, and
q is an integer of from 1 to 10, preferably 1 or 2.

6. The method according to claim 5, **characterized in that**, in the epoxide of formula (III),
q is 1 or 2, and
when q is 2, R₂ is -CH₂-O-C₁₋₁₀-alkylenyl-O-CH₂-, and
when q is 1, R₂ is -CH₂-O-C₁₋₁₀-alkyl.

7. The method according to one of claims 1 to 4, **characterized in that** the cyclic carbonate is an ethylene carbonate of formula (IV) 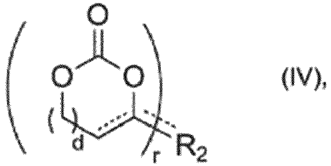 where R₂ is an optionally divalent or polyvalent, linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom, optionally comprising at least one -OH group, or a (poly)alkylene glycol, in particular a (poly)alkylene glycol of the formula -O-[CHRₐCH₂O]_{b}-R_{b}, where Rₐ is H or a C₁₋₄ alkyl radical, R_{b} is -H and b is from 1 to 100, even more preferably a C₁₋₁₀ hydroxyalkyl,
------ a single or double bond, preferably a single bond,
d is 0 or 1, preferably 0, and
r is an integer of from 1 to 10, preferably 1 or 2.

8. The method according to one of claims 3 to 7, **characterized in that**
(i) X₁ is -NH₂ or a derivative thereof, and
q or r is 1;
(ii) X₁ is -OH or a derivative thereof, and
q or r is 2.

9. The method according to claim 1, in which the alkenyl ether polyol is prepared by reacting an alkenyl ether, containing at least one alkenyl ether group and at least one functional group selected from (i) epoxide groups and (ii) cyclic carbonate groups or derivatives thereof, with an alcohol, thiol, a carboxylic acid, or an amine or derivatives thereof, **characterized in that** the alkenyl ether polyol is an alkenyl ether polyol of formula (V) 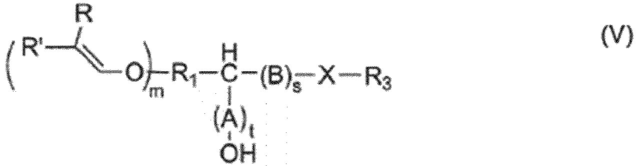 where
R₁ is an at least divalent linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom,
R₃ is an optionally divalent or polyvalent, linear or branched, substituted or unsubstituted alkyl having from 1 to 20 carbon atoms, linear or branched, substituted or unsubstituted heteroalkyl having from 1 to 20 carbon atoms and at least one oxygen or nitrogen atom, or a (poly)alkylene glycol of the formula -O-[CHRₐCH₂O]_{b}-R_{b}, where Rₐ is H or a C₁₋₄ alkyl radical, R_{b} is H or 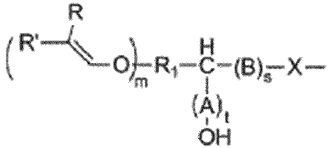 and b is from 1 to 100;
X is O, S, OC(=O), OC(=O)O, OC(=O)OC(=O), NR_{z}, NR_{z}C(=O)O, NR_{z}C(=O)NR_{z} or OC(=O)NR_{z},
each R and R' is selected independently from H, C₁₋₂₀ alkyl, C₂₋₂₀ alkenyl, in particular one of R and R' being H and the other being C₁₋₄ alkyl or both R and R' being H,
each A and B is independently selected from CR"R''',
R" and R'" are selected independently from H, a functional group, an organic radical, in particular H and C₁₋₂₀ alkyl, or R" and R'" together or with the carbon atom to which they are bonded are an organic radical, or two of R" and R'" that are bonded to adjacent carbon atoms together form a bond in order to form a double bond between the adjacent carbon atoms, m is an integer of from 1 to 10, preferably 1,
s and t are each 0 or an integer of from 1 to 10, where s + t = 1 or more, in particular 1 or 2, and
R_{z} is H or 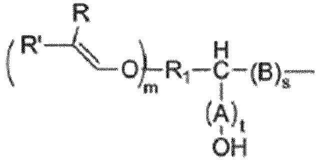 where, if X is not NR_{z}, with R_{z} = 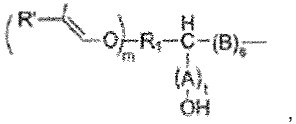 R₃ comprises at least one substituent that is selected from -OH and

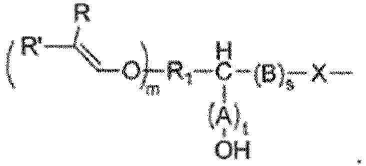

10. The method according to claim 9, **characterized in that** the alkenyl ether, which contains at least one alkenyl ether group and at least one functional group selected from (i) epoxide groups and (ii) cyclic carbonate groups or derivatives thereof, is an alkenyl ether of formula (VI) or (VII) 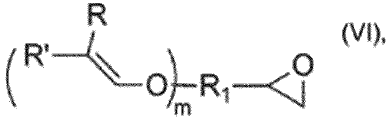 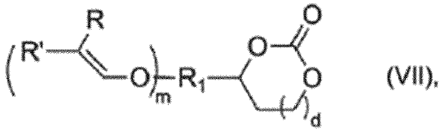 where R₁ is an at least divalent linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom,
each R and R' is selected independently from H, C₁₋₂₀ alkyl and C₂₋₂₀ alkenyl, in particular one of R and R' being H and the other being C₁₋₄ alkyl or both R and R' being H,
d is 0 or 1, preferably 0, and
m is an integer from 1 to 10, preferably 1.

11. The method according to claim 10, **characterized in that**, in the alkenyl ethers of formula (VI) or (VII),
R₁ is -C₁₋₁₀-alkylenyl-O-CH₂-.

12. The method according to claim 10 or 11, **characterized in that** the alkenyl ether is reacted with an alcohol, the alcohol being a diol or polyol or a corresponding alcoholate, in particular a polyalkelene glycol of the formula HO-[CHRₐCH₂O]_{b}-H, where Rₐ is H or a C₁₋₄ alkyl radical and b is from 1 to 100, in particular from 1 to 10.

13. An alkenyl ether polyol of formula (I) or (V) 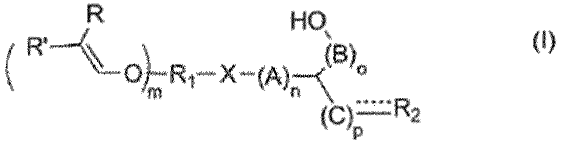 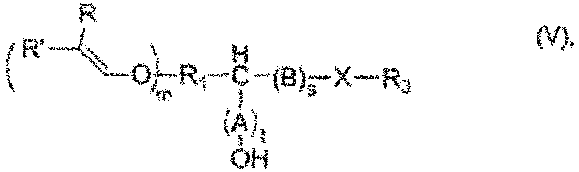 where R₁ is an at least divalent linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom,
R₂ is an optionally divalent or polyvalent, linear or branched, substituted or unsubstituted alkyl having from 1 to 20 carbon atoms or linear or branched, substituted or unsubstituted heteroalkyl having from 1 to 20 carbon atoms and at least one oxygen or nitrogen atom, optionally comprising at least one -OH group, or a (poly)alkylene glycol, in particular a (poly)alkylene glycol of the formula -O-[CHRₐCH₂O]_{b}-R_{b}, where Rₐ is H or a C₁₋₄ alkyl radical, R_{b} is -H and b is from 1 to 100,
R₃ is an optionally divalent or polyvalent, linear or branched, substituted or unsubstituted alkyl having from 1 to 20 carbon atoms, linear or branched, substituted or unsubstituted heteroalkyl having from 1 to 20 carbon atoms and at least one oxygen or nitrogen atom, or a (poly)alkylene glycol of the formula -O-[CHRₐCH₂O]_{b}-R_{b}, where Rₐ is H or a C₁₋₄ alkyl radical, Rb is -H or 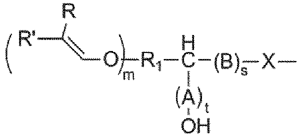 and b is from 1 to 100,
in formula (I), X is O, S, C(=O)O, OC(=O)O, C(=O)OC(=O)O, NRₓ, NRₓC(=O)O, NRₓC(=O)NRₓ or OC(=O)NRₓ,
in formula (V), X is O, S, OC(=O), OC(=O)O, OC(=O)OC(=O), NR_{z}, NR_{z}C(=O)O, NR_{z}C(=O)NR_{z} or OC(=O)NR_{z},
each R and R' is selected independently from H, C₁₋₂₀ alkyl and C₂₋₂₀ alkenyl, in particular one of R and R' being H and the other being C₁₋₄ alkyl or both R and R' being H,
each A, B and C is selected independently from CR"R''',
R" and R'" are selected independently from H, a functional group and an organic radical, in particular H and C₁₋₂₀ alkyl, or R" and R'" together or with the carbon atom to which they are bonded are an organic radical, or two of R" and R'" that are bonded to adjacent carbon atoms together form a bond in order to form a double bond between the adjacent carbon atoms,
------ is a single or double bond, and, if it is a double bond,
the C that is bonded to R₂ bears only one substituent R" or R''',
m is an integer of from 1 to 10, preferably 1,
n, p and o are each 0 or an integer of from 1 to 10, where n + p + o = 1 or more, in particular 1 or 2,
s and t are each 0 or an integer of from 1 to 10, where s + t = 1 or more, in particular 1 or 2,
Rₓ is H, or 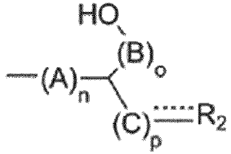 where, if X is not NRₓ, with Rₓ = 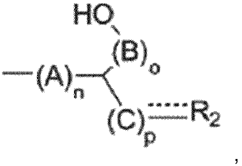 R₂ comprises at least one substituent, which is selected from and 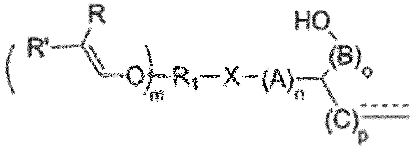 R_{z} is H or 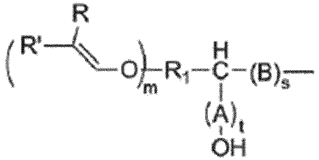 where, if X is not NR_{z}, with R_{z} = 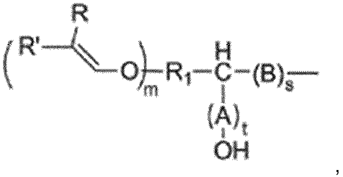 R₃ comprises at least one substituent that is selected from -OH and

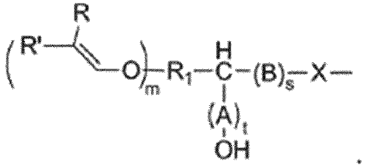

14. The alkenyl ether polyol according to claim 13, **characterized in that** it can be obtained by a method according to one or more of claims 1 to 12.

15. The use of an alkenyl ether polyol according to claim 13 or 14 for synthesizing radiation-crosslinkable oligomers or polymers by means of polyaddition or polycondensation reactions, in particular for synthesizing UV- and EB-curable polyesters, polyethers, polyurethanes and polyureas, particularly preferably UV-curable polyurethanes.

16. A UV-curable polyurethane polymer that can be obtained by reacting at least one alkenyl ether polyol according to claim 13 or 14 with a polyisocyanate.

## Revendications

1. Procédé de production d'un polyol d'éther alcénylique contenant au moins un groupe éther alcénylique, en particulier un groupe éther 1-alcénylique, et au moins deux groupes hydroxyle (-OH), par
A) mise en réaction d'un éther alcénylique contenant au moins un groupe éther alcénylique et au moins un groupe fonctionnel choisi parmi -OH, -COOH, -SH, -NH₂ et leurs dérivés,
avec (i) un époxy ou (ii) un carbonate cyclique ou un dérivé de celui-ci ; ou
B) mise en réaction d'un éther alcénylique contenant au moins un groupe éther alcénylique et au moins un groupe fonctionnel choisi parmi (i) les groupes époxy, et (ii) les groupes carbonate cycliques ou leurs dérivés,
avec un alcool, un thiol, un acide carboxylique ou une amine, ou les dérivés de ceux-ci.

2. Procédé selon la revendication 1, dans lequel le polyol d'éther alcénylique est produit par mise en réaction d'un éther alcénylique contenant au moins un groupe éther alcénylique et au moins un groupe fonctionnel choisi parmi -OH, -COOH, -SH, -NH₂ et leurs dérivés, avec (i) un époxy ou (ii) un carbonate cyclique ou un dérivé de celui-ci, **caractérisé en ce que** le polyol d'éther alcénylique est un polyol d'éther alcénylique de Formule (I) 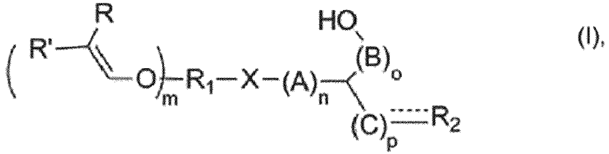 dans laquelle
R₁ représente un groupe alkyle au moins bivalent, linéaire ou ramifié, substitué ou non-substitué, ayant de 1 à 20 atomes de carbone, ou un groupe hétéro-alkyle linéaire ou ramifié,
substitué ou non-substitué, ayant de 1 à 20 atomes de carbone et au moins un atome d'oxygène ou d'azote,
R₂ représente un groupe alkyle, éventuellement bivalent ou multivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote, chacun étant éventuellement avec au moins un groupe -OH, ou un (poly)alkylèneglycol, en particulier un (poly)alkylèneglycol de Formule - O-[CHRₐCH₂O]_{b}-R_{b}, dans laquelle Rₐ représente H ou un radical alkyle en C₁₋₄, R_{b} représente -H, et b se situe dans la plage allant de 1 à 100,
X représente O, S, C(=O)O, OC(=O)O, C(=O)OC(=O)O, NRₓ, NRₓC(=O)O, NRₓC(=O)NRₓ ou OC(=O)NRₓ,
chacun de R et R' est, indépendamment, choisi parmi H, un groupe alkyle en C₁₋₂₀ et un groupe alcényle en C₂₋₂₀, où en particulier l'un de R et R' représente H et l'autre représente un groupe alkyle en C₁₋₄ ou les deux représentent H,
chacun de A, B et C est, indépendamment, choisi parmi CR"R''',
R" et R''' sont, indépendamment, choisis parmi H, un groupe fonctionnel, et un radical organique, en particulier H et un groupe alkyle en C₁₋₂₀, ou R" et R''' forment ensemble, ou avec l'atome de carbone auquel ils sont liés, un radical organique, ou deux de R" et R''', qui sont liés à des atomes de carbone adjacents, forment ensemble une liaison pour former une double liaison entre les atomes de carbone adjacents,
------ représente une liaison simple ou double, dans laquelle, lorsqu'il s'agit d'une double liaison,
l'atome de carbone lié à R₂ ne porte qu'un substituant R" ou R''',
m est un nombre entier de 1 à 10, de préférence 1,
n, p et o sont chacun égaux à 0 ou sont un nombre entier de 1 à 10, où n+p+o = 1 ou plus, en particulier 1 ou 2, et
Rₓ représente H, ou 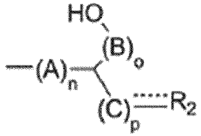 où, lorsque X ne représente pas NRₓ avec Rₓ = 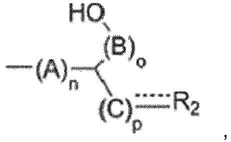 R2 présente au moins un substituant choisi parmi -OH et

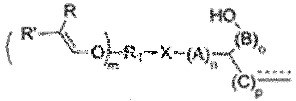

3. Procédé selon la revendication 2, **caractérisé en ce que** l'éther alcénylique, qui contient au moins un groupe éther alcénylique et au moins un groupe fonctionnel choisi parmi -OH, - COOH, -SH, -NH₂ et leurs dérivés, est un éther alcénylique de Formule (II) 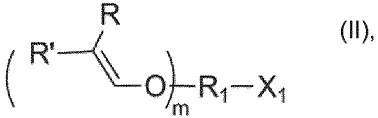 dans laquelle
R₁ représente un groupe alkyle au moins bivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote,
X₁ représente un groupe fonctionnel choisi parmi -OH, -COOH, -SH, -NHR_{y} et leurs dérivés, R_{y} représente H ou un radical organique,
chacun de R et R' est, indépendamment, choisi parmi H, un groupe alkyle en C₁₋₂₀ et un groupe alcényle en C₂₋₂₀, où en particulier l'un de R et R' représente H et l'autre représente un groupe alkyle en C₁₋₄ ou les deux représentent H, et
m est un nombre entier de 1 à 10, de préférence 1.

4. Procédé selon la revendication 3, **caractérisé en ce que**, dans l'éther alcénylique de Formule (II)
m est égal à 1,
X₁ représente -OH ou -NH₂, de préférence -OH,
R₁ est un radical alkyle en C₁₋₁₀ bivalent, linéaire ou ramifié, en particulier éthyle, propyle, butyle, pentyle ou hexyle, et
l'un de R et R' représente H et l'autre représente H ou -CH₃.

5. Procédé selon l'une quelconque des revendications 1 à 4, **caractérisé en ce que** l'époxy est un époxy de Formule (III) 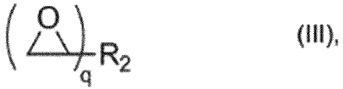 dans laquelle R₂ représente un groupe alkyle, éventuellement bivalent ou multivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote, éventuellement avec au moins un groupe -OH, ou un (poly)alkylèneglycol, en particulier un (poly)alkylèneglycol de Formule -O-[CHRₐCH₂O]_{b}-R_{b}, dans laquelle Rₐ représente H ou un radical alkyle en C₁₋₄, R_{b} représente - H, et b se situe dans la plage allant de 1 à 100, et
q est un nombre entier de 1 à 10, de préférence 1 ou 2.

6. Procédé selon la revendication 5, **caractérisé en ce que**, dans l'époxy de Formule (III)
q est égal à 1 ou 2, et
lorsque q est égal à 2, R₂ représente -CH₂-O-C₁₋₁₀ alkylényle-O-CH₂-, et
lorsque q est égal à 1, R₂ représente -CH₂-O-C₁₋₁₀ alkyle.

7. Procédé selon l'une quelconque des revendications 1 à 4, **caractérisé en ce que** le carbonate cyclique est un carbonate d'éthylène de Formule (IV) 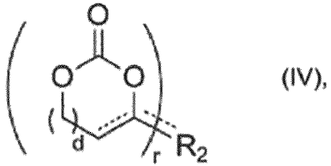 dans laquelle R₂ représente un groupe alkyle, éventuellement bivalent ou multivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote, éventuellement avec au moins un groupe -OH, ou un (poly)alkylèneglycol, en particulier un (poly)alkylèneglycol de Formule -O-[CHRₐCH₂O]_{b}-R_{b}, dans laquelle Rₐ représente H ou un radical alkyle en C₁₋₄, R_{b} représente - H, et b se situe dans la plage allant de 1 à 100, et de façon encore plus préférée un groupe hydroxyalkyle en C₁₋₁₀,
------ représente une liaison simple ou double, de préférence une liaison simple,
d est égal à 0 ou 1, de préférence à 0, et
r est un nombre entier de 1 à 10, de préférence 1 ou 2.

8. Procédé selon l'une des revendications 3 à 7, **caractérisé, en ce que**
(i) X₁ représente -NH₂ ou l'un de ses dérivés, et
q ou r est égal à 1 ;
(ii) X₁ représente -OH ou l'un de ses dérivés, et
q ou r est égal à 2.

9. Procédé selon la revendication 1, dans lequel le polyol d'éther alcénylique est produit par mise en réaction d'un éther alcénylique contenant au moins un groupe éther alcénylique et au moins un groupe fonctionnel choisi parmi (i) les groupes époxy, et (ii) les groupes carbonate cycliques ou leurs dérivés, avec un alcool, un thiol, un acide carboxylique ou une amine, ou les dérivés de ceux-ci, **caractérisé en ce que** le polyol d'éther alcénylique est un polyol d'éther alcénylique de Formule (V) 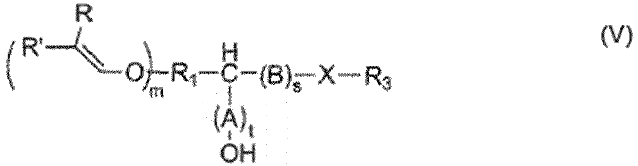 dans laquelle
R₁ représente un groupe alkyle au moins bivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote,
R₃ représente un groupe alkyle, éventuellement bivalent ou multivalent, linéaire ou ramifié, substitué ou non-substitué, ayant de 1 à 20 atomes de carbone, un groupe hétéro-alkyle linéaire ou ramifié, substitué ou non-substitué, ayant de 1 à 20 atomes de carbone et au moins un atome d'oxygène ou d'azote, ou un (poly)alkylèneglycol de Formule -O-[CHRₐCH₂O]_{b}-R_{b}, dans laquelle Rₐ représente H ou un radical alkyle en C₁₋₄, R_{b} représente H ou 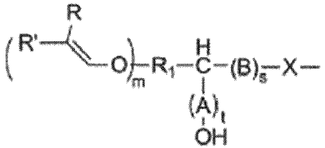 et b se situe dans la plage allant de 1 à 100 ;
X représente O, S, OC(=O), OC(=O)O, OC(=O)OC(=O), NR_{z}, NR_{z}C(=O)O, NR_{z}C(=O)NR_{z} ou OC(=O)NR_{z},
chacun de R et R' est, indépendamment, choisi parmi H, un groupe alkyle en C₁₋₂₀ et un groupe alcényle en C₂₋₂₀, où en particulier l'un de R et R' représente H et l'autre représente un groupe alkyle en C₁₋₄ ou les deux représentent H,
chacun de A et B est, indépendamment, choisi parmi CR"R"",
R" et R"" sont, indépendamment, choisis parmi H, un groupe fonctionnel, et un radical organique, en particulier H et un groupe alkyle en C₁₋₂₀, ou R" et R"" forment ensemble, ou avec l'atome de carbone auquel ils sont liés, un radical organique, ou deux de R" et R"", qui sont liés à des atomes de carbone adjacents, forment ensemble une liaison pour former une double liaison entre les atomes de carbone adjacents, m est un nombre entier de 1 à 10, de préférence 1,
s et t sont chacun égaux à 0 ou sont un nombre entier de 1 à 10, où s+t = 1 ou plus, en particulier 1 ou 2, et
R_{z} représente H, ou 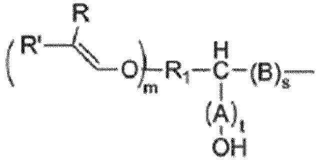 où, lorsque X ne représente pas NR_{z} avec R_{z} = 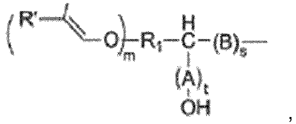 R₃ présente au moins un substituant choisi parmi -OH et

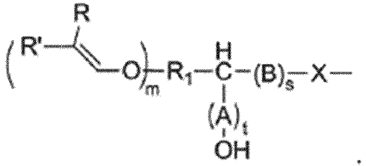

10. Procédé selon la revendication 9, **caractérisé en ce que** l'éther alcénylique, qui contient au moins un groupe éther alcénylique et au moins un groupe fonctionnel choisi parmi (i) les groupes époxy, et (ii) les groupes carbonate cycliques ou leurs dérivés, est un éther alcénylique de Formule (VI) ou (VII) 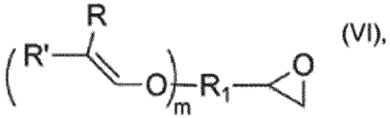 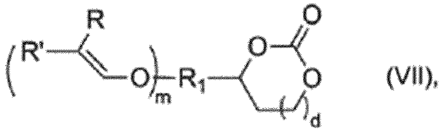 dans lesquelles R₁ représente un groupe alkyle au moins bivalent linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote,
chacun de R et R' est, indépendamment, choisi parmi H, un groupe alkyle en C₁₋₂₀ et un groupe alcényle en C₂₋₂₀, où en particulier l'un de R et R' représente H et l'autre représente un groupe alkyle en C₁₋₄ ou les deux représentent H,
d est égal à 0 ou 1, de préférence à 0, et
m est un nombre entier de 1 à 10, de préférence 1.

11. Procédé selon la revendication 10, **caractérisé en ce que**, dans les éthers alcényliques de Formule (VI) ou (VII)
R₁ représente -C₁₋₁₀-alkylényle-O-CH₂-.

12. Procédé selon la revendication 10 ou 11, **caractérisé en ce que** l'éther alcénylique est mis à réagir avec un alcool, dans lequel l'alcool est un diol ou un polyol ou un alcoolate correspondant, en particulier un polyalkylèneglycol de formule HO-[CHRₐCH₂O]_{b}H, dans lequel Rₐ représente H ou un radical alkyle en C₁₋₄ et b se situe dans la plage allant de 1 à 100, en particulier de 1 à 10.

13. Polyol d'éther alcénylique de formule (I) ou (V) 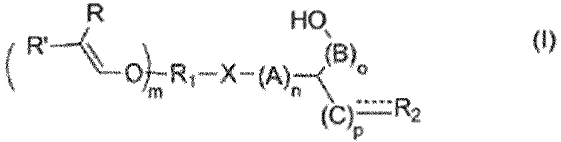 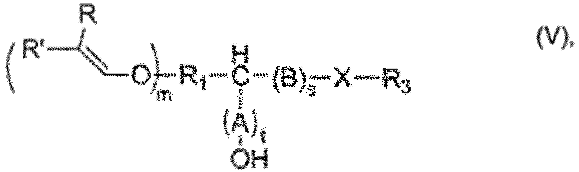 dans lesquelles
R₁ représente un groupe alkyle au moins bivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote,
R₂ représente un groupe alkyle, éventuellement bivalent ou multivalent, linéaire ou ramifié, substitué ou non-substitué, ayant de 1 à 20 atomes de carbone, ou un groupe hétéro-alkyle linéaire ou ramifié, substitué ou non-substitué, ayant de 1 à 20 atomes de carbone et au moins un atome d'oxygène ou d'azote, éventuellement avec au moins un groupe -OH, ou un (poly)alkylèneglycol, en particulier un (poly)alkylèneglycol de Formule -O-[CHRₐCH₂O]_{b}-R_{b}, dans laquelle Rₐ représente H ou un radical alkyle en C₁₋₄, R_{b} représente -H, et b se situe dans la plage allant de 1 à 100,
R₃ représente un groupe alkyle, éventuellement bivalent ou multivalent, linéaire ou ramifié, substitué ou non-substitué, ayant de 1 à 20 atomes de carbone, un groupe hétéro-alkyle linéaire ou ramifié, substitué ou non-substitué, ayant de 1 à 20 atomes de carbone et au moins un atome d'oxygène ou d'azote, ou un (poly)alkylèneglycol de Formule -O-[CHRₐCH₂O]_{b}-R_{b}, dans laquelle Rₐ représente H ou un radical alkyle en C₁₋₄, R_{b} représente -H ou 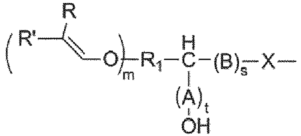 et b se situe dans la plage allant de 1 à 100,
dans la Formule (I), X représente O, S, C(=O)O, OC(=O)O, C(=O)OC(=O)O, NRₓ, NRₓC(=O)O, NRₓC(=O)NRₓ ou OC(=O)NRₓ,
dans la Formule (V), X représente O, S, OC(=O), OC(=O)O, OC(=O)OC(=O), NRz, NR_{z}C(=O)O, NR_{z}C(=O)NR_{z}, ou OC(=O)NR_{z},
chacun de R et R' est, indépendamment, choisi parmi H, un groupe alkyle en C₁₋₂₀ et un groupe alcényle en C₂₋₂₀, où en particulier l'un de R et R' représente H et l'autre représente un groupe alkyle en C₁₋₄ ou les deux représentent H,
chacun de A, B et C est, indépendamment, choisi parmi CR"R''',
R" et R"" sont, indépendamment, choisis parmi H, un groupe fonctionnel, et un radical organique, en particulier H et un groupe alkyle en C₁₋₂₀, ou R" et R''' forment ensemble, ou avec l'atome de carbone auquel ils sont liés, un radical organique, ou deux de R" et R''', qui sont liés à des atomes de carbone adjacents, forment ensemble une liaison pour former une double liaison entre les atomes de carbone adjacents,
représente une liaison simple ou double, dans laquelle, lorsqu'il s'agit d'une ------ double liaison,
le C qui est lié à R₂ ne porte qu'un substituant R" ou R''',
m est un nombre entier de 1 à 10, de préférence 1,
n, p et o sont chacun égaux à 0 ou sont un nombre entier de 1 à 10, où n+p+o = 1 ou plus, en particulier 1 ou 2,
s et t sont chacun égaux à 0 ou sont un nombre entier de 1 à 10, où s+t = 1 ou plus, en particulier 1 ou 2,
Rₓ représente H, ou 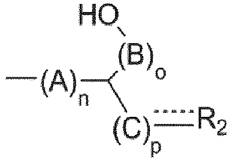 où, lorsque X ne représente pas NRₓ avec Rₓ = 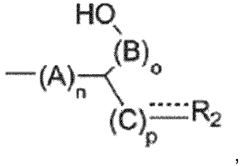 R2 présente au moins un substituant choisi parmi et 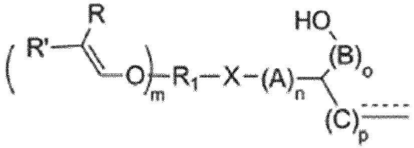 R_{z} représente H, ou 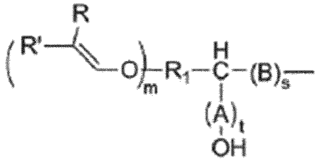 où, lorsque X ne représente pas Nr_{z} avec R_{z} = 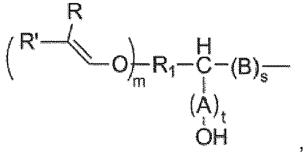 R₃ présente au moins un substituant choisi parmi -OH et

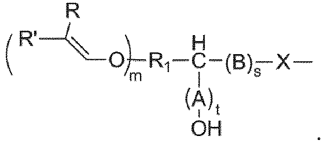

14. Polyol d'éther alcénylique selon la revendication 13, **caractérisé en ce qu'**il peut être obtenu par un procédé selon l'une ou plusieurs des revendications 1 à 12.

15. Utilisation d'un polyol d'éther alcénylique selon la revendication 13 ou 14 pour la synthèse d'oligomères ou de polymères réticulables par rayonnement au moyen de réactions de polyaddition ou de polycondensation, en particulier pour la synthèse de polyesters, polyéthers, polyuréthanes et polyurées durcissables par ultraviolets ou faisceau électronique, de façon particulièrement préférée de polyuréthanes durcissables aux ultraviolets ou par faisceau électronique.

16. Polymère de polyuréthane durcissable aux ultraviolets pouvant être obtenu par mise en réaction d'au moins un polyol d'éther alcénylique selon la revendication 13 ou 14 avec un poly-isocyanate.
